# EUROPÄISCHE PATENTSCHRIFT

(11) **EP 2 743 267 B1**
(45) Veröffentlichungstag und Bekanntmachung des Hinweises auf die Patenterteilung: **04.03.2015**
(21) Anmeldenummer: 12197372.1
(22) Anmeldetag: 14.12.2012
(51) Int. Cl.: C07D 403/14, A61K 6/087

(54) **Auf polymerisierbaren Alkinen und Aziden basierende Dentalmassen**
Dental materials based on polymerisable azides and alkenes
Matières dentaires à base d'alcynes et d'azides polymérisables

(43) Veröffentlichungstag der Anmeldung: 18.06.2014
(73) Patentinhaber: Ivoclar Vivadent AG, 9494 Schaan (LI)
(72) Erfinder: Moszner, Norbert, 9493 Mauren (LI); Burtscher, Dr. Peter, 6830 Rankweil (AT); Fischer, Urs Karl, 9320 Arbon (CH); Hirt, Dr. Thomas, 9445 Rebstein (CH); Schubert, Prof. Dr. Ulrich S., 07743 Jena (DE); Hager, Dr. Martin, 07743 Jena (DE); Happ, Bobby, 07745 Jena (DE); Sandmann, Benedict, 07743 Jena (DE)
(74) Vertreter: UEXKÜLL & STOLBERG

(56) Entgegenhaltungen:
- EP-A1- 2 455 059
- LUTZ, J. F.: "1,3-dipolar cycloadditions of azides and alkynes: a universal ligation tool in polymer and materials science", ANGEWANDTE CHEMIE, INTERNATIONAL EDITION, Bd. 46, Nr. 7, 9. Januar 2007 (2007-01-09) , Seiten 1018-1025, XP002500213, WILEY VCH VERLAG, WEINHEIM; DE ISSN: 1433-7851, DOI: 10.1002/ANIE.200604050
- BANERT, K. ET AL.: "The Exciting Chemistry of Tetraazidomethane", ANGEWANDTE CHEMIE, INTERNATIONAL EDITION., Bd. 46, 4. Dezember 2006 (2006-12-04), XP002696721, DEVCH VERLAG, WEINHEIM; DE ISSN: 0570-0833, DOI: 10.1002/ANIE.200603960

## Beschreibung

Die vorliegende Erfindung betrifft thermisch- und/oder lichthärtende Dentalwerkstoffe und insbesondere dentale Kompositmaterialien mit hervorragenden mechanischen Eigenschaften zur Herstellung von dentalen Kompositen für Inlays, Onlays, Kronen, Brücken oder Verblendmaterialien.

Dentale Komposite, die z.B. als direktes Füllungsmaterial, Inlay, Onlay, Kronen oder Verblendwerkstoff eingesetzt werden, bestehen in der Regel aus einer polymerisierbaren organischen Matrix und aus einem oder mehreren Füllstoffen. Je nach Art der Füllstoffe, der Monomermatrix und der Anwendung kann der Füllgrad zwischen ca. 50-90 Gew.-% variieren. Die Komposite werden üblicherweise nach der Partikelgrösse und Zusammensetzung der Füllstoffe unterteilt in Makrofüller-Komposite, homogene und heterogene Mikrofüller-Komposite und Hybrid-Komposite (F. Lutz, R. W. Phillips, A classification and evaluation of composite resin systems, J. Prosthet. Dent. 50 (1983) 480-488).

Die polymerisierbare organische Matrix besteht üblicherweise vor allem aus einer Mischung von Monomeren, Initiator-Komponenten, Stabilisatoren und Pigmenten (J. Viohl, K. Dermann, D. Quast, S. Venz, Die Chemie zahnärztlicher Füllungskunststoffe, Carl Hanser Verlag, München-Wien 1986, 21-27). Dabei werden als Harze meist Mischungen von Dimethacrylaten eingesetzt (vgl. A. Peutzfeldt, Resin composites in dentistry: the monomer systems, Eur. J. Oral. Sci. 105 (1997) 97-116; J. W. Nicolson, H. M. Anstice, The chemistry of modern dental filling materials, J. Chem Ed. 76 (1999) 1497-1501; J. W. Stansburry, Curing dental resins and composites by photopolymerization, J. Esthet. Dent., 12 (2000) 300-308; N. Moszner, T. Hirt, New Polymer-Chemical Developments in Clinical Dental Polymer Materials: Enamel-Dentin Adhesives and Restorative Composites, J. Polym. Sci. Part A: Polym. Chem. 50 (2012) 4369-4402). Beispiele hierfür sind die hochviskosen Dimethacrylate 2,2-Bis[4-(2-hydroxy-3-methacryloyloxypropyl)phenyl]-propan (Bis-GMA) und 1,6-Bis-[2-methacryloyloxyethoxycarbonylamino]-2,4,4-trimethylhexan (UDMA) und die als Verdünnermonomere genutzten niedriger viskosen Dimethacrylate wie Bismethacryloyloxymethyl-tricyclo[5.2.1]decan (TCDMA), Decandiol-1,10-dimethacrylate (D₃MA) und Triethylenglycoldimethacrylat (TEGDMA).

Die Aushärtung der bekannten Dimethacrylat-basierenden dentalen Komposite kann durch thermische, redoxinitiierte oder lichtinduzierte radikalische Polymerisation unter Verwendung geeigneter Initiatoren erfolgen. Es hat sich allerdings als nachteilig herausgestellt, dass es bei diesen Kompositen meist schon innerhalb von Sekunden und bei entsprechend geringem Monomerumsatz am sogenannten Gelpunkt zur Bildung eines Polymernetzwerks kommt, in dem Füllerpartikel fest eingebunden sind. Das fertige 3-dimensionale Polymernetzwerk enthält auch bei nahezu vollständigem Monomerumsatz zahlreiche nichtumgesetzte Doppelbindungen. Daher sind die mit den bekannten Dimethacrylat-basierenden dentalen Kompositen erzielbaren Vernetzungsdichten und somit auch die erreichbaren mechanischen Eigenschaften stark begrenzt.

Der Erfindung liegt daher die Aufgabe zugrunde, Dentalwerkstoffe bereitzustellen, die sich durch höheren Umsatz der polyreaktionsfähigen Gruppen, verbesserte mechanische Eigenschaften und vor allem durch einen erhöhten Elastizitätsmodul auszeichnen und sich insbesondere zur Herstellung von dentalen Kompositen für Inlays, Onlays, Kronen, Brücken oder Verblendmaterialien eignen.

Diese Aufgabe wird erfindungsgemäss durch einen Dentalwerkstoff auf Basis mindestens einer Verbindung der Formel I gelöst

A-[X-Q-(Y-CG)ₙ]ₘ Formel I,

in der
- A: für -O-, -N=, -NR³-, =N-N=, -NR³-N=, =N-NR³-, -NR³-NR³- oder einen m-wertigen Alkylen-Rest, Cycloalkylen-Rest, Heterocycloalkylen-Rest, Arylen-Rest, Heteroarylen-Rest, gemischten Alkylen-Cyclyl-Rest wie Alkylen-Cyclyl-Rest, Cyclyl-Alkylen-Rest, Cyclyl-Alkylen-Cyclyl-Rest oder Alkylen-Cyclyl-Alkylen-Cyclyl-Alkylen-Rest oder Cyclyl-O-Cyclyl-Rest steht, wobei Alkylen jeweils unabhängig für einen linearen oder verzweigten aliphatischen C₁-C₅₀-Rest steht, der durch ein oder mehrere -O-, -S-, -NR³-, -CO-O-, -O-CO-, -O-CO-O-, -CO-NR³-, -NR³-CO-, -O-CO-NR³-, -NR³-CO-O- oder -NR³-CO-NR³- unterbrochen sein kann, Cycloalkylen jeweils unabhängig für einen cycloaliphatischen C₃-C₁₈-Rest steht,
Heterocycloalkylen jeweils unabhängig für einen heterocycloaliphatischen C₃-C₁₈-Rest steht,
Arylen jeweils unabhängig für einen aromatischen C₆-C₁₈-Rest steht,
Heteroarylen jeweils unabhängig für einen heteroaromatischen C₃-C₁₈-Rest steht,
Cyclyl jeweils unabhängig für Cycloalkylen, Heterocycloalkylen, Arylen oder Heteroarylen steht und
die genannten Reste jeweils unabhängig einen oder mehrere Substituenten, insbesondere ausgewählt aus C₁-C₅-Alkyl, OH, OCH₃ und OCOCH₃, tragen können,
- CG: jeweils unabhängig für eine Azidgruppe N₃ oder eine Alkingruppe ausgewählt aus der Gruppe bestehend aus -CR¹R²-C≡CH, 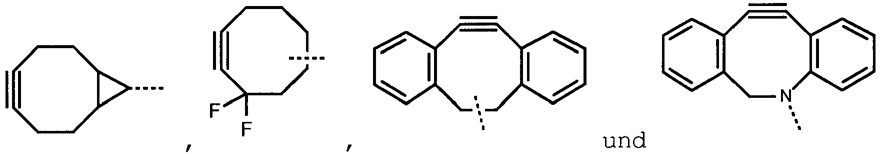 steht,
mit der Massgabe, dass das Dentalwerkstoff mindestens eine Verbindung der Formel I, die eine Azidgruppe aufweist, und mindestens eine Verbindung der Formel I enthält, die eine Alkingruppe aufweist,
- Q: jeweils unabhängig entfällt oder für einen (n+1)-wertigen linearen oder verzweigten aliphatischen C₁-C₂₀-Alkylen-Rest, der durch ein oder mehrere -O- oder -S- unterbrochen sein kann, oder einen Phenylenrest steht, wobei die genannten Reste jeweils unabhängig einen oder mehrere Substituenten, insbesondere ausgewählt aus CH₃, C₂H₅, OH, OCH₃ und OCOCH₃, tragen können,
- R¹, R² und R³: jeweils unabhängig für H oder einen C₁-C₁₀-Alkylrest stehen,
- X und Y: jeweils unabhängig entfallen oder für -O-, -S-, -NR³-, -CO-, -CO-O-, -O-CO-, -O-CO-O-, -CO-NR³-, -NR³-CO-, -O-CO-NR³-, -NR³-CO-O- oder -NR³-CO-NR³- stehen,
- m: jeweils unabhängig die Werte 2 bis 6 annehmen kann und
- n: jeweils unabhängig die Werte 1 bis 4 annehmen kann.

Der Hinweis, dass ein Rest durch eine Gruppe wie beispielsweise -O-unterbrochen sein kann, ist so zu verstehen, dass die Gruppe in die Kohlenstoffkette des Restes eingeschoben wird, d.h. beidseitig von Kohlenstoffatomen begrenzt wird. Die Anzahl dieser Gruppen ist daher um mindestens 1 kleiner als die Zahl der Kohlenstoffatome, und die Gruppen können nicht endständig sein. Erfindungsgemäss sind Reste, die nicht durch die genannten Gruppen unterbrochen sind, bevorzugt.

Erfindungsgemäss werden nur solche Verbindungen in Erwägung gezogen, die in mit der chemischen Valenzlehre vereinbar sind.

Besonders bevorzugt sind solche Verbindungen der Formel I, bei denen jeweils unabhängig voneinander
- A: für -N= oder einen m-wertigen Alkylen-Rest, Cycloalkylen-Rest, Heterocycloalkylen-Rest, Arylen-Rest, Heteroarylen-Rest, gemischten Alkylen-Cyclyl-Rest wie Alkylen-Cyclyl-Rest, Cyclyl-Alkylen-Rest, Cyclyl-Alkylen-Cyclyl-Rest oder Alkylen-Cyclyl-Alkylen-Cyclyl-Alkylen-Rest oder Cyclyl-O-Cyclyl-Rest steht, wobei Alkylen jeweils unabhängig für einen linearen oder verzweigten aliphatischen C₁-C₂₀-Rest steht, der durch ein oder mehrere -O-, -S-, -CO-O-, -O-CO- oder -O-CO-O- unterbrochen sein kann, Cycloalkylen jeweils unabhängig für einen cycloaliphatischen C₃-C₁₂-Rest steht,
Heterocycloalkylen jeweils unabhängig für einen heterocycloaliphatischen C₃-C₁₂-Rest steht,
Arylen jeweils unabhängig für einen aromatischen C₆-C₁₂-Rest steht,
Heteroarylen jeweils unabhängig für einen heteroaromatischen C₃-C₁₂-Rest steht,
Cyclyl jeweils unabhängig für Cycloalkylen, Heterocycloalkylen, Arylen oder Heteroarylen steht und
die genannten Reste jeweils unabhängig einen oder mehrere Substituenten, insbesondere ausgewählt aus C₁-C₃-Alkyl, OCH₃ und OCOCH₃, tragen können,
- CG: jeweils unabhängig für eine Azidgruppe N₃ oder eine Alkingruppe ausgewählt aus der Gruppe bestehend aus -CR¹R²-C≡CH, 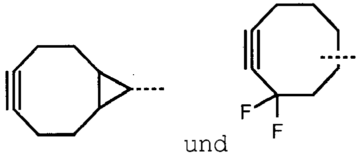 steht,
- Q: jeweils unabhängig entfällt oder für einen (n+1)-wertigen linearen oder verzweigten aliphatischen C₁-C₁₀-Alkylen-Rest, der durch ein oder mehrere -O- oder -S- unterbrochen sein kann, oder einen Phenylenrest steht, wobei die genannten Reste jeweils unabhängig einen oder mehrere Substituenten, insbesondere ausgewählt aus CH₃, C₂H₅, OCH₃ und OCOCH₃, tragen können,
- R¹, R² und R³: jeweils unabhängig für H oder einen C₁-C₅-Alkylrest und insbesondere einen C₁-C₃-Alkylrest stehen,
- X und Y: jeweils unabhängig entfallen oder für -O-, -S-, -CO-O-, -O-CO-, -O-CO-O-, -CO-NR³-, -NR³-CO-, -O-CO-NR³-, -NR³-CO-O- oder -NR³-CO-NR³- stehen,
- m: jeweils unabhängig die Werte 2, 3 oder 4 annehmen kann,
- n: jeweils unabhängig die Werte 1, 2 oder 3 und insbesondere 1 oder 2 annehmen kann und am meisten bevorzugt 1 ist.

Besonders bevorzugt sind dabei Verbindungen, in denen alle Variablen eine der oben definierten Bedeutungen und insbesondere eine der bevorzugten Bedeutungen haben.

Erfindungsgemäss besonders bevorzugt sind Dentalwerkstoffe, die mindestens ein Azid der Formel IA

A-[X-Q-(Y-CG)ₙ]ₘ Formel IA,

in der
- A: für -O-, -N=, -NR³-, =N-N=, -NR³-N=, =N-NR³-, -NR³-NR³- oder einen m-wertigen Alkylen-Rest, Cycloalkylen-Rest, Heterocycloalkylen-Rest, Arylen-Rest, Heteroarylen-Rest, gemischten Alkylen-Cyclyl-Rest wie Alkylen-Cyclyl-Rest, Cyclyl-Alkylen-Rest, Cyclyl-Alkylen-Cyclyl-Rest oder Alkylen-Cyclyl-Alkylen-Cyclyl-Alkylen-Rest oder Cyclyl-O-Cyclyl-Rest steht, wobei Alkylen jeweils unabhängig für einen linearen oder verzweigten aliphatischen C₁-C₅₀-Rest steht, der durch ein oder mehrere -O-, -S-, -NR³-, -CO-O-, -O-CO-, -O-CO-O-, -CO-NR³-, -NR³-CO-, -O-CO-NR³-, -NR³-CO-O- oder -NR³-CO-NR³- unterbrochen sein kann,
Cycloalkylen jeweils unabhängig für einen cycloaliphatischen C₃-C₁₈-Rest steht,
Heterocycloalkylen jeweils unabhängig für einen heterocycloaliphatischen C₃-C₁₈-Rest steht,
Arylen jeweils unabhängig für einen aromatischen C₆-C₁₈-Rest steht,
Heteroarylen jeweils unabhängig für einen heteroaromatischen C₃-C₁₈-Rest steht,
Cyclyl jeweils unabhängig für Cycloalkylen, Heterocycloalkylen, Arylen oder Heteroarylen steht und
die genannten Reste jeweils unabhängig einen oder mehrere Substituenten, insbesondere ausgewählt aus C₁-C₅-Alkyl, OH, OCH₃ und OCOCH₃, tragen können,
- CG: jeweils unabhängig für eine Azidgruppe N₃ steht,
- Q: jeweils unabhängig entfällt oder für einen (n+1)-wertigen linearen oder verzweigten aliphatischen C₁-C₁₀-Alkylen-Rest, der durch ein oder mehrere -O- oder -S- unterbrochen sein kann, oder einen Phenylenrest steht, wobei die genannten Reste jeweils unabhängig einen oder mehrere Substituenten, insbesondere ausgewählt aus CH₃, C₂H₅, OCH₃ und OCOCH₃, tragen können,
- R¹, R² und R³: jeweils unabhängig für H oder einen C₁-C₁₀-Alkylrest stehen,
- X und Y: jeweils unabhängig entfallen oder für -O-, -S-, -NR³-, -CO-, -CO-O-, -O-CO-, -O-CO-O-, -CO-NR³-, -NR³-CO-, -O-CO-NR³-, -NR³-CO-O- oder -NR³-CO-NR³- stehen,
- m: jeweils unabhängig die Werte 2 bis 6 annehmen kann und
- n: jeweils unabhängig die Werte 1 bis 4 annehmen kann, und
mindestens ein Alkin der Formel IB enthalten

A-[X-Q-(Y-CG)ₙ]ₘ Formel IB,

in der
- A: für -O-, -N=, -NR³-, =N-N=, -NR³-N=, =N-NR³-, -NR³-NR³- oder einen m-wertigen Alkylen-Rest, Cycloalkylen-Rest, Heterocycloalkylen-Rest, Arylen-Rest, Heteroarylen-Rest, gemischten Alkylen-Cyclyl-Rest wie Alkylen-Cyclyl-Rest, Cyclyl-Alkylen-Rest, Cyclyl-Alkylen-Cyclyl-Rest oder Alkylen-Cyclyl-Alkylen-Cyclyl-Alkylen-Rest oder Cyclyl-O-Cyclyl-Rest steht, wobei Alkylen jeweils unabhängig für einen linearen oder verzweigten aliphatischen C₁-C₅₀-Rest steht, der durch ein oder mehrere -O-, -S-, -NR³-, -CO-O-, -O-CO-, -O-CO-O-, -CO-NR³-, -NR³-CO-, -O-CO-NR³-, -NR³-CO-O- oder -NR³-CO-NR³- unterbrochen sein kann,
Cycloalkylen jeweils unabhängig für einen cycloaliphatischen C₃-C₁₈-Rest steht,
Heterocycloalkylen jeweils unabhängig für einen heterocycloaliphatischen C₃-C₁₈-Rest steht,
Arylen jeweils unabhängig für einen aromatischen C₆-C₁₈-Rest steht,
Heteroarylen jeweils unabhängig für einen heteroaromatischen C₃-C₁₈-Rest steht,
Cyclyl jeweils unabhängig für Cycloalkylen, Heterocycloalkylen, Arylen oder Heteroarylen steht und
die genannten Reste jeweils unabhängig einen oder mehrere Substituenten, insbesondere ausgewählt aus C₁-C₅-Alkyl, OH, OCH₃ und OCOCH₃, tragen können,
- CG: jeweils unabhängig für eine Alkingruppe ausgewählt aus der Gruppe bestehend aus -CR¹R²-C≡CH, 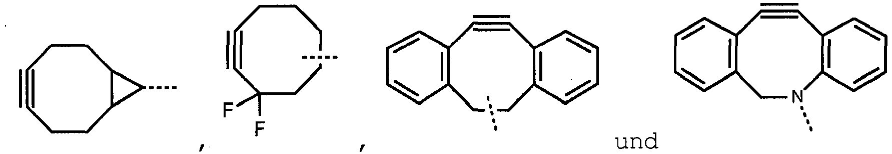 steht,
- Q: jeweils unabhängig entfällt oder für einen (n+1)-wertigen linearen oder verzweigten aliphatischen C₁-C₁₀-Alkylen-Rest, der durch ein oder mehrere -O- oder -S- unterbrochen sein kann, oder einen Phenylenrest steht, wobei die genannten Reste jeweils unabhängig einen oder mehrere Substituenten, insbesondere ausgewählt aus CH₃, C₂H₅, OCH₃ und OCOCH₃, tragen können,
- R¹, R² und R³: jeweils unabhängig für H oder einen C₁-C₁₀-Alkylrest stehen,
- X und Y: jeweils unabhängig entfallen oder für -O-, -S-, -NR³-, -CO-, -CO-O-, -O-CO-, -O-CO-O-, -CO-NR³-, -NR³-CO-, -O-CO-NR³-, -NR³-CO-O- oder -NR³-CO-NR³- stehen,
- m: jeweils unabhängig die Werte 2 bis 6 annehmen kann und
- n: jeweils unabhängig die Werte 1 bis 4 annehmen kann.

Dabei sind solche Verbindungen der Formel IA und IB besonders bevorzugt, in der jeweils unabhängig voneinander eine oder mehrere der Variablen eine der oben für Formel I definierten bevorzugten Bedeutungen haben. Besonders bevorzugt sind Verbindungen, in denen alle Variablen eine der oben definierten Bedeutungen und insbesondere eine der bevorzugten Bedeutungen haben.

Ausserdem sind Dentalwerkstoffe bevorzugt, in denen Alkingruppen und Azidgruppen ganz oder annähernd im stöchiometrischen Verhältnis vorliegen und insbesondere im Verhältnis 2:1 - 1:2, vorzugsweise im Verhältnis 1,5:1 - 1:1,5, besonders bevorzugt im Verhältnis 1,1:1 - 1:1,1 und am meisten bevorzugt im Verhältnis 1,05:1 - 1:1,05 vorliegen.

Weiter bevorzugt sind solche Dentalwerkstoffe, in denen die Verbindungen der Formel I eine mittlere Funktionalität von > 2,2 und insbesondere > 2,5 aufweisen. Unter der mittleren Funktionalität wird die durchschnittliche Anzahl an Gruppen CG bezogen auf die in dem Dentalwerkstoff enthaltenen Moleküle von Verbindungen der Formel I verstanden.

Es wurde überraschend gefunden, dass die erfindungsgemässen Dentalwerkstoffe, die mindestens eine Verbindung der Formel I, und insbesondere mindestens ein Azid der Formel IA und mindestens ein Alkin der Formel IB enthalten, hervorragend polymerisierbar sind und einen sehr hohen Umsatz der polyreaktionsfähigen Gruppen zeigen und nach der Aushärtung verbesserte mechanische Eigenschaften wie einen deutlich erhöhten Elastizitätsmodul aufweisen.

Darüber hinaus hat sich gezeigt, dass auf Basis der erfindungsgemässen Dentalwerkstoffe durch geeignete Auswahl der Verbindungen der Formel I, und insbesondere der multifunktionellen Azide der Formel IA und der multifunktionellen Alkine der Formel IB, sowie durch Variation der mittleren Funktionalität dieser Verbindungen in dem Dentalwerkstoff durch Einstellung eines geeigneten Mischungsverhältnis insbesondere der Azide und Alkine, Polymernetzwerke mit massgeschneiderten Eigenschaften hergestellt werden können. Dabei nimmt die Netzwerkdichte mit der mittleren Funktionalität der Verbindungen und dem erreichten Umsatz der polymerisierbaren Gruppen zu. Durch Variation der Struktur der Verbindungen der Formel I, wie etwa die Verwendung von flexiblen oder steifen Spacern zur Anbindung der Azid- bzw. Alkin-Gruppen, lassen sich die Eigenschaften der gebildeten Polycycloadditionsnetzwerke weiter beeinflussen. Ausserdem können durch thermische Nachbehandlung der Umsetzungsgrad der funktionellen Gruppen und damit die Vernetzungsdichte zusätzlich erhöht werden. Schliesslich lassen sich die mechanischen Eigenschaften durch Zugabe von Füllstoffen weiter verbessern. Die erfindungsgemässen Dentalwerkstoffe auf Basis von Verbindungen der Formel I eignen sich daher insbesondere für die Herstellung von dentalen Restaurationsmaterialien wie hochmoduligen Komposit-Frässkörpern für CAD-CAM-basierte Verarbeitungstechniken zur Herstellung von zahnfarbenen Inlays, Onlays, Brücken oder Kronen.

Verbindungen der Formel I lassen sich nach an sich bekannten Synthesemethoden einfach herstellen. So sind Azide der Formel IA beispielsweise durch nucleophile Substitution von geeigneten di- oder höherfunktionalisierten Halogeniden (Z=Cl,Br,I) oder p-Toluolsulfonsäureestern (Z=OTos) mit Natriumazid NaN₃ zugänglich:

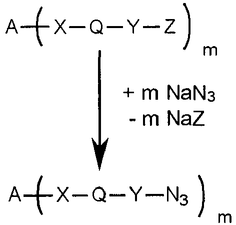

### Konkretes Beispiel: Umsetzung von 2,2,2-Tri(chlormethyl)ethanol mit Natriumazid

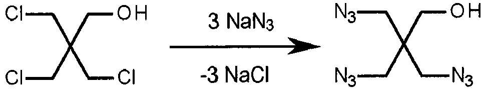

Weiterhin lassen sich Azidgruppen in an sich bekannter Weise aus aromatischen Hydrazinen durch Diazotierung, aus Alkoholen durch Mitsunobo-Reaktion oder aus Isocyanaten durch Addition von 2-Azido-ethanol herstellen.

### Konkretes Beispiel: Umsetzung von Trimethylhexamethylendiisocyanat mit 2-Azidoethanol oder mit 2-Bromethanol gefolgt von Natriumazid

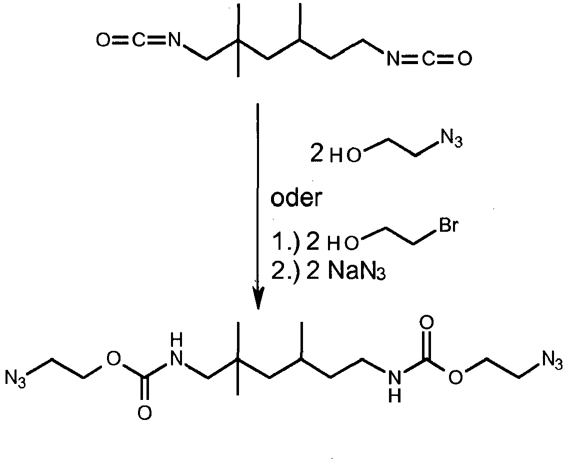

Alkine der Formel IB sind ausgehend von kommerziell erhältlichen monofunktionellen Alkinen, wie Propargylamin (Z=NH₂), Propargylessigsäure (Z=CH₂COOH), Propargylalkohol (Z=OH) oder Propargylhalogeniden (Z=Cl, Br) leicht durch Umsetzung mit geeigneten di- oder höherfunktionalisierten Verbindungen wie Di- oder Polyolen zugänglich:

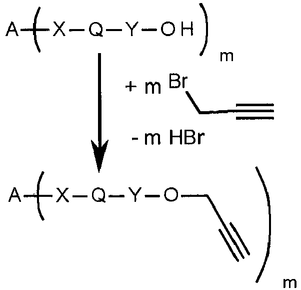

### Konkretes Beispiel: Umsetzung von Tetra(hydroxymethyl)methan mit Propargylbromid

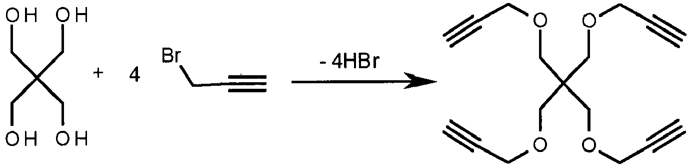

Die Synthese von Aziden der Formel IB mit Cyclooctingruppen kann beispielsweise durch Umsetzung von bi- oder höherfunktionellen Isocyanaten mit entsprechenden Hydroxycyclooctinen erfolgen.

### Konkretes Beispiel: Umsetzung von Trimethylhexamethylendiisocyanat mit Bicyclo[6.1.0]non-4-in-9-ol

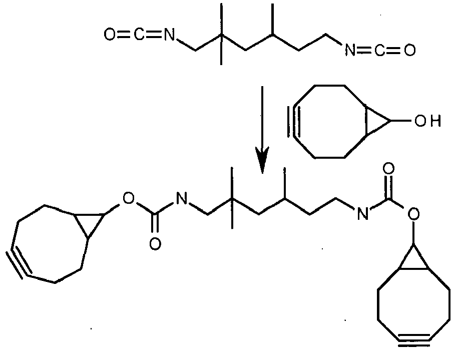

Beispiele für die erfindungsgemässen Verbindungen der Formel I sind:

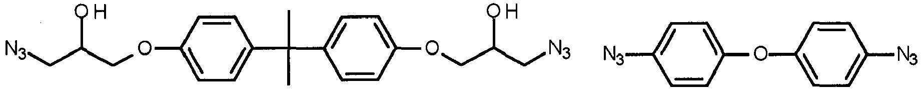

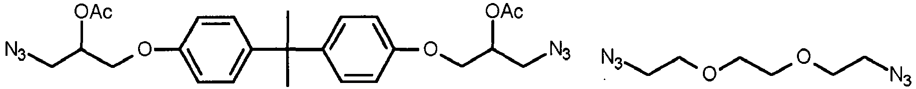

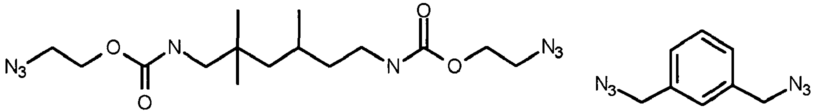

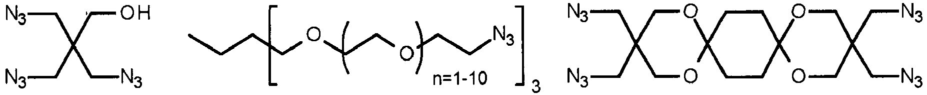

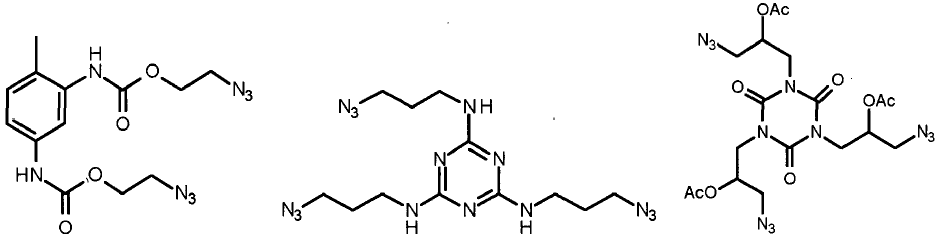

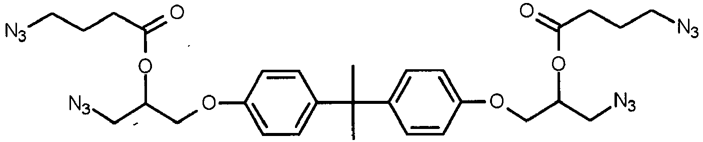

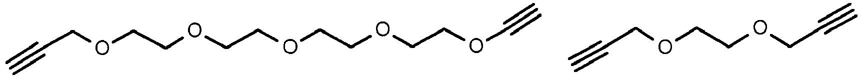

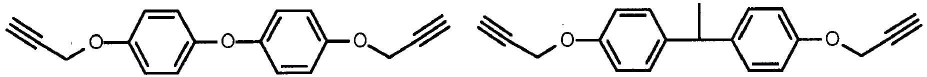

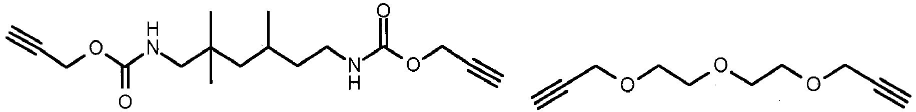

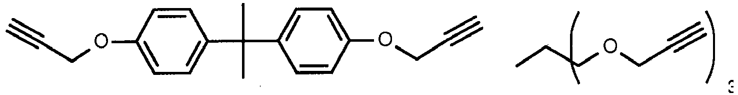

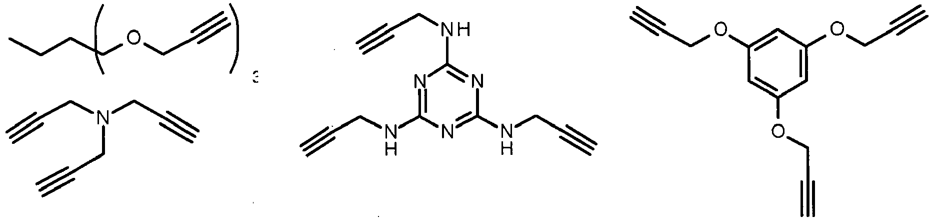

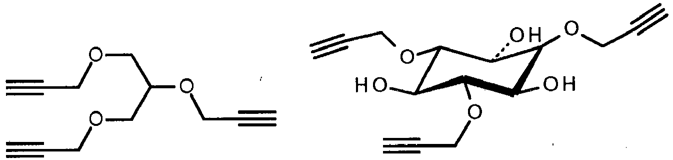

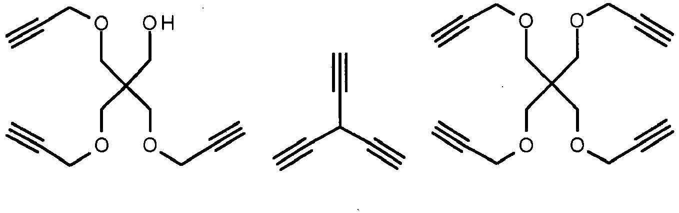

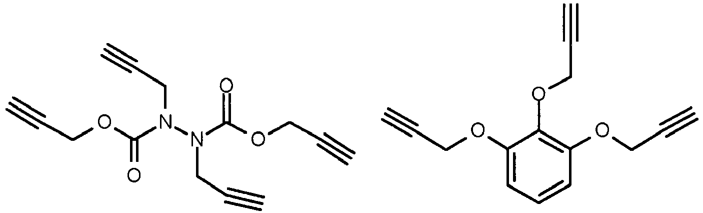

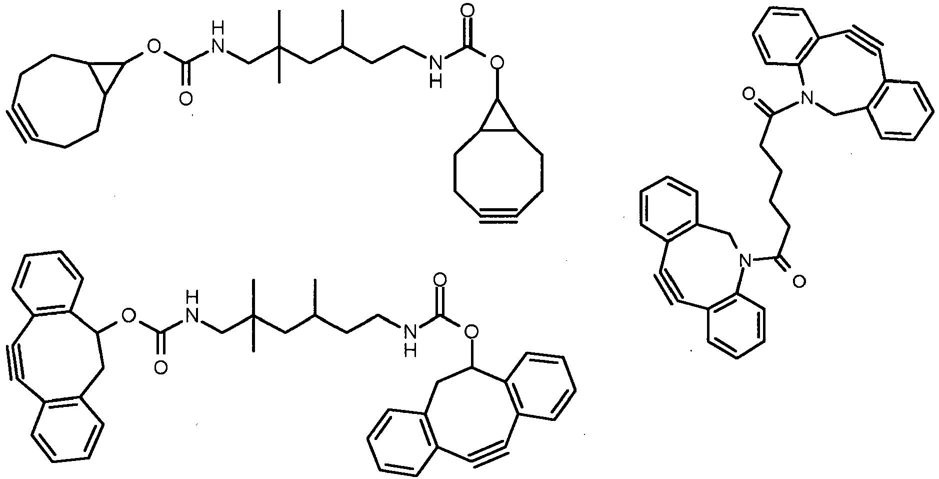

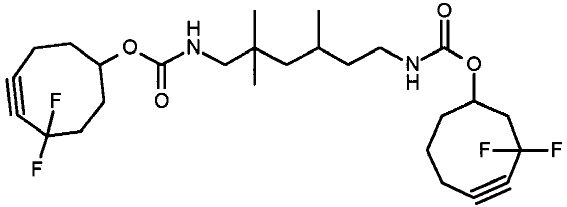

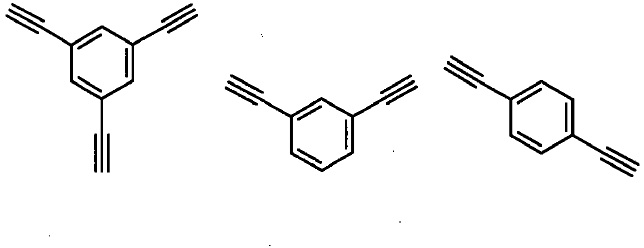

Die erfindungsgemässen Dentalwerkstoffe enthalten vorzugsweise auch einen Katalysator für die Azid-Alkin-Cycloaddition.

Als thermische Katalysatoren für die Azid-Alkin-Cycloaddition eignen sich vor allem Kupfer-, Ruthenium-, Nickel-, Palladium- und Platin-Verbindungen und -Komplexe und insbesondere Kupfer(I)-, Ruthenium(I)-, Nickel(II)-, Palladium(II)- und Platin(II)-Verbindungen und -Komplexe. Besonders geeignet sind einfache Kupfer(I)-salze wie CuCl, CuBr, CuI oder CuOAc und Kupfer(I)-Komplexe wie CuOTf, Cu(CH₃C(O)CHC(O)CH₃), CuI·P(OEt)₃, [Cu(CH₃CN)₄]PF₆ oder Cu(PPh₃)Br. Diese können zusätzlich durch chelatisierende Liganden wie Tris-[(1-benzyl-1H-1,2,3-triazol-4-yl)-methyl]-amin (TBTA), N,N,N',N",N"-Pentamethyldiethylentriamin (PMDETA), N,N,N'-Trimethylethylendiamin (TRMEDA) oder Bathophenanthrolindisulfonate (Batho) stabilisiert werden. Weiterhin lassen sich Mischungen aus Kupfer(II)-salzen mit Reduktionsmitteln verwenden. Beispiele für geeignete Kupfer(II)-Salze sind CuSO₄, CuCl₂, CuBr₂ und CuI₂. Diese lassen sich etwa mit Ascorbinsäure oder deren Natriumsalz in die entsprechenden Cu(I)-Spezies überführen. Alternativ können katalytische Cu(I)-Spezies aus Kupfer(II)-Salzen auch elektrochemisch erzeugt werden. Weitere Beispiele für geeignete Verbindungen sind Cp*Ru(PPh₃)₂Cl, [Cp*RuCl]ₙ und eine Mischung aus PtCl₂ und PMDETA. CuOAc ist besonders bevorzugt. Eine besondere Katalysatoraktivität kann mit einer Mischung von Tripropargylamin und CuOAc erreicht werden, wobei das CuOAc vorzugsweise in einer Menge von 0,15-0,25 mol% eingesetzt wird. Dabei kann die Bildung der aktiven Katalysatorspezies durch die im Laufe der Reaktion eintretende Farbänderung von grün zu gelb verfolgt werden.

Als lichtinduzierbare Katalysatoren für die Azid-Alkin-Cycloaddition eignen sich vor allem photoreduzierbare Kupfer(II)-Verbindungen sowie Mischungen von Kupfer(II)-Verbindungen mit lichtinduzierbaren Reduktionsmitteln. Beispiele für geeignete Kupfer(II)-Verbindungen sind CuSO₄, CuCl₂, CuBr₂, Cu(OH)₂, Cu(OAc)₂, CuO, Cu(ClO₄)₂ und Cu(NO₃)₂ sowie deren Hydrate und THF-Addukte. Geeignete lichtinduzierbare Reduktionsmittel insbesondere für den UVA-Bereich (320-400 nm) sind vor allem Norrish-Typ-I-Photoinitiatoren wie Benzophenon, 2-Hydroxy-2-methyl-1-phenyl-1-propanon, 4-(2-Hydroxyethoxy)-phenyl-(2-hydroxy-2-propyl)-keton, Bis-(2,6-dimethoxybenzoyl)-2,4,4-trimethylpentyl-phosphinoxid, Bis-(2,4,6-trimethylbenzoyl)-phenylphosphinoxid, 2,2-Di-methoxy-2-phenylacetophenon, 2-Benzyl-2-dimethylamino-4'-morpholino-butyrophenon (DBMP), 2,4,6-(Trimethylbenzoyl)-diphenylphosphinoxid (TMDPO) und Diphenyliodoniumhexafluorophosphat. Geeignete lichtinduzierbare Reduktionsmittel insbesondere für den sichtbaren Bereich (400-800 nm) sind vor allem Photoinitiatoren aus der Gruppe der Titanocene, wie Dicyclopentadienyl-bis-[2,6-difluoro-3-(1-pyrrolyl)-phenyl]-titanium, und der α-Diketone, wie 9,10-Phenanthrenchinon, 1-Phenyl-propan-1,2-dion, Diacetyl oder Campherchinon. Diese lichtinduzierbaren Reduktionsmittel können etwa in Kombination mit CuCl₂/PMDETA blaulichtinduziert katalytische Cu(I)-Spezies für die Azid-Alkin-Cycloaddition bilden.
Photoinitiatoren als lichtinduzierbare Reduktionsmittel für den sichtbaren Bereich bilden bei Bestrahlung zudem polymerisationsauslösende Radikale, gegebenenfalls in Gegenwart von Co-Initiatoren. Beispiele hierfür sind Mischungen von Campherchinon mit tertiären Aminen, insbesondere tertiären aromatischen Aminen. Besonders geeignete tertiäre aromatische Aminen sind N,N-Dialkyl-aniline wie N,N-Dimethylanilin, N,N-Dialkyl-p-toluidine wie N-Dimethyl-p-toluidin, N,N-Dialkyl-3,5-xylidine wie N,N,3,5-Tetramethylanilin, p-(N,N-Dialkylamino)-phenylethanole, p-(N,N-Dialkylamino)-benzoesäure-derivate wie p-(Dimethylamino)-benzonitril, p-(N,N-Dialkylamino)-benzaldehyde wie N,N-Dimethylamino-p-benzaldehyd, p-(N,N-Dialkyl-amino)-phenylessigsäureester wie p-(Dimethylamino)-benzoesäureethylester oder p-(N,N-Dialkylamino)-phenylpropionsäureestern. Geeignet sind auch tertiäre aliphatische Amine wie Tri-n-butylamin, Dimethylaminoethan-2-ol, Triethanolamin, Dimethylaminoethylmethacrylat oder N,N-Dimethylbenzylamin.

Beim Einsatz von substituierten Cycloalkinen wie difluorierten Cyclooctinen kann häufig auf die Verwendung von Katalysatoren verzichtet werden, da in diesem Fall die Azid-Alkin-Cycloaddition unter Abbau der Ringspannung stattfindet.

Besonders geeignet sind auch Hybridmaterialien, die neben mindestens einer Verbindung der Formel I, und insbesondere mindestens einem Azid der Formel IA und mindestens einem Alkin der Formel IB, radikalisch polymerisierbare Monomere enthalten. Solche Hybridmaterialien können durch Katalysatorkombinationen von Kupfer(I)- oder Kupfer(II)-Verbindungen mit radikalischen Photoinitiatoren als lichtinduzierbaren Reduktionsmitteln stufenweise (erst thermisch und dann lichtinduziert) oder simultan (lichtinduziert) ausgehärtet werden.

Als radikalisch polymerisierbare Monomere (Co-Monomere) sind insbesondere mono- oder polyfunktionelle (Meth)acrylsäurederivate geeignet. Unter monofunktionellen (Meth)acrylsäurederivaten werden Verbindungen mit einer, unter polyfunktionellen (Meth)acrylsäure-derivaten Verbindungen mit zwei oder mehr, vorzugsweise 2 bis 4 (Meth)acrylsäuregruppen verstanden. Polyfunktionelle Monomere haben eine vernetzende Wirkung.

Erfindungsgemäss bevorzugte mono- oder polyfunktionelle (Meth)acrylsäurederivate sind Methyl-, Ethyl-, Hydroxyethyl-, Butyl-, Benzyl-, Tetrahydrofurfuryl- oder Isobornyl(meth)acrylat, p-Cumyl-phenoxyethylenglycolmethacrylat (CMP-1E), Bisphenol-A-di(meth)acrylat, Bis-GMA (ein Additionsprodukt aus Methacrylsäure und Bisphenol-A-diglycidylether), ethoxy- oder propoxyliertes Bisphenol-A-Dimethacrylat, wie etwa das Bisphenol-A-Dimethacrylat SR-348c (Sartomer) mit 3 Ethoxygruppen oder 2,2-Bis-[4-(2-methacryloxypropoxy)-phenyl]-propan, UDMA (ein Additionsprodukt aus 2-Hydroxyethylmethacrylat (HEMA) und 2,2,4-Trimethylhexamethylendiisocyanat), Di-, Tri- oder Tetraethylenglycoldi(meth)acrylat, Trimethylolpropantri(meth)acrylat, Pentaerythrittetra(meth)acrylat, Glycerindi- und -tri(meth)acrylat, 1,4-Butandioldi(meth)acrylat, 1,10-Decandioldi(meth)acrylat (D₃MA) und 1,12-Dodecandioldi(meth)acrylat.

Besonders bevorzugte mono- oder polyfunktionelle (Meth)acrylsäurederivate sind N-mono- oder -disubstitiuierte Acrylamide wie N-Ethylacrylamid, N,N-Dimethacrylamid, N-(2-Hydroxyethyl)acrylamid oder N-Methyl-N-(2-hydroxyethyl)acrylamid, N-monosubstituierte Methacrylamide wie N-Ethylmethacrylamid oder N-(2-Hydroxyethyl)-methacrylamid sowie N-Vinylpyrrolidon und Allylether. Diese Monomere zeichnen sich durch eine hohe Hydrolysestabilität aus und eignen sich aufgrund ihrer relativ geringen Viskosität besonders als Verdünnermonomere.

Bevorzugte polyfunktionelle (Meth)acrylsäurederivate mit hoher Hydrolysestabilität sind vernetzende Pyrrolidone wie 1,6-Bis-(3-vinyl-2-pyrrolidonyl)-hexan, Bisacrylamide wie Methylen- oder Ethylenbisacrylamid und Bis(meth)acrylamide wie N,N'-Diethyl-1,3-bis-(acrylamido)-propan, 1,3-Bis(methacrylamido)-propan, 1,4-Bis-(acrylamido)-butan oder 1,4-Bis(acryloyl)-piperazin, die durch Umsetzung aus den entsprechenden Diaminen mit (Meth)acrylsäurechlorid synthetisiert werden können.

Vorzugsweise werden Mischungen der vorstehend genannten Monomere verwendet.

Die erfindungsgemässen Dentalwerkstoffe können neben mindestens einer Verbindung der Formel I, und insbesondere mindestens einer Verbindung der Formel IA und mindstens einer Verbindung der Formel IB, und ggf. den oben genannten Co-Monomeren vorzugweise auch zusätzliche radikalisch polymerisierbare, säuregruppenhaltige Monomere (Haftmonomere) enthalten. Bevorzugte Säuregruppen sind Carbonsäuregruppen, Phosphonsäuregruppen, Phosphorsäuregruppen und Sulfonsäuregruppen.

Bevorzugte Monomere mit polymerisierbaren Carbonsäuren sind Maleinsäure, Acrylsäure, Methacrylsäure, 2-(Hydroxymethyl)acrylsäure, 4-(Meth)acryloyloxyethyltrimellitsäureanhydrid, 10-Methacryloyloxydecylmalonsäure, N-(2-Hydroxy-3-methacryloyloxypropyl)-N-phenylglycin und 4-Vinylbenzoesäure.

Bevorzugte Monomere mit polymerisierbaren Phosphonsäuregruppen sind Vinylphosphonsäure, 4-Vinylphenylphosphonsäure, 4-Vinylbenzylphosphonsäure, 2-Methacryloyloxyethylphosphonsäure, 2-Methacrylamidoethylphosphonsäure, 4-Methacrylamido-4-methyl-pentylphosphonsäure, 2-[4-(Dihydroxyphosphoryl)-2-oxa-butyl]-acrylsäure, 2-[4-(Dihydroxyphosphoryl)-2-oxa-butyl]-acrylsäureethyl- und -2,4,6-trimethylphenylester.

Bevorzugte Monomere mit polymerisierbaren Phosphorsäuregruppen sind 2-Methacryloyloxypropylmono- oder -dihydrogenphosphat, 2-Methacryloyloxyethylmono- oder -dihydrogenphosphat, 2-Methacryloyloxyethylphenylhydrogenphosphat, Dipentaerythritolpentamethacryloyloxyphosphat, 10-Methacryloyloxydecyl-dihydrogenphosphat, Phosphorsäuremono-(1-acryloyl-piperidin-4-yl)-ester, 6-(Methacrylamido)-hexyldihydrogenphosphat und 1,3-Bis-(N-acryloyl-N-propyl-amino)-propan-2-yl-dihydrogenphosphat. Bevorzugte Monomere mit polymerisierbaren Sulfonsäuregruppen sind Vinylsulfonsäure, 4-Vinylphenylsulfonsäure und 3-(Methacrylamido)-propylsulfonsäure.

Weiterhin enthalten die erfindungsgemässen Zusammensetzungen zur Verbesserung der mechanischen Eigenschaften oder zur Einstellung der Viskosität vorzugsweise auch organische oder anorganische Füllstoffpartikel.

Bevorzugte anorganische partikuläre Füllstoffe sind amorphe kugelförmige Materialien auf Basis von Oxiden wie ZrO₂ und TiO₂ oder Mischoxiden aus SiO₂, ZrO₂ und/oder TiO₂ mit einer mittleren durchschnittlichen Partikelgrösse von 0,005 bis 2 µm, vorzugsweise 0,1 bis 1 µm, nanopartikuläre oder mikrofeine Füllstoffe wie pyrogene Kieselsäure oder Fällungskieselsäure mit einer mittleren durchschnittlichen Partikelgrösse von 5 bis 200 nm, vorzugsweise 10 bis 100 nm, Minifüllstoffe wie Quarz-, Glaskeramik- oder Glaspulver beispielsweise von Barium- oder Strontiumaluminiumsilikatgläsern mit einer durchschnittlichen Teilchengrösse von 0,01 bis 15 µm, vorzugsweise 0,1 bis 1 µm, sowie röntgenopake Füllstoffe wie Ytterbiumtrifluorid oder nanopartikuläres Tantal(V)-oxid bzw. Bariumsulfat oder Mischoxide von SiO₂ mit Ytterbium(III)-oxid oder Tantal(V)-oxid mit einer mittleren durchschnittlichen Partikelgrösse von 10 bis 1000 nm, vorzugsweise 100 bis 300 nm. Die Bestimmung der durchschnittlichen Partikelgrösse kann insbesondere durch Transmissions- oder Rasterelektronenmikroskopie oder durch Laserbeugung erfolgen. Vorzugsweise kann im Bereich von 1 nm bis 50 nm die Transmissionselektronenmikroskopie, im Bereich von 50 nm bis 1 µm die Rasterelektronenmikroskopie und im Bereich von 1 µm bis 100 µm die Laserbeugung zur Bestimmung der durchschnittlichen Partikelgrösse verwendet werden. Geeignet sind auch faserförmige Füllstoffe, Nanofasern oder Wiskers.

Zur Verbesserung des Verbundes zwischen den Füllstoffpartikeln und der Polycycloadditionsmatrix können insbesondere Füllstoffe auf Basis von SiO₂ mit Azid- oder Alkin-funktionalisierten Silanen oberflächenmodifiziert werden. Beispiele für solche Silane sind 3-Azidopropyltriethoxysilan und N-[3-(Triethoxysilyl)-propyl]-carbaminsäurepropargylester. Zur Oberflächenmodifizierung von nichtsilikatischen Füllstoffen etwa auf Basis von ZrO₂ oder TiO₂ können auch funktionalisierte saure Phosphate wie Propargyldihydrogenphosphat eingesetzt werden.

Ausserdem können die erfindungsgemässen Dentalwerkstoffe weitere Additive enthalten, vor allem Stabilisatoren, Aromastoffe, Farbmittel, mikrobiozide Wirkstoffe, fluoridionenabgebende Additive, optische Aufheller, Weichmacher, UV-Absorber oder Lösungsmittel wie Wasser oder Ethanol bzw. entsprechende Lösungsmittelgemische.

Besonders bevorzugt sind Dentalwerkstoffe auf Basis mindestens einer Verbindung der Formel I, und insbesondere mindestens eines Azids der Formel IA und mindestens eines Alkins der Formel IB, welche die folgenden Bestandteile enthalten:
a) 0,1 bis 80 Gew.-%, insbesondere 1 bis 60 Gew.-%, bevorzugt 5 bis 50 Gew.-% und besonders bevorzugt 10 bis 40 Gew.-% mindestens einer Verbindung der Formel I und vorzugsweise einer Mischung mindestens eines Azids der Formel IA und mindestens eines Alkins der Formel IB,
b) 0,01 bis 10 Gew.-%, bevorzugt 0,1 bis 3 Gew.-% und besonders bevorzugt 0,2 bis 2 Gew.-% Katalysator,
c) 0 bis 80 Gew.-%, bevorzugt 1 bis 60 Gew.-% und besonders bevorzugt 5 bis 50 Gew.-% Co-Monomer,
d) 0 bis 90 Gew.-%, bevorzugt 10 bis 80 Gew.-% und besonders bevorzugt 20 bis 70 Gew.-% Füllstoff und
e) 0 bis 70 Gew.-%, bevorzugt 1 bis 50 Gew.-% und besonders bevorzugt 5 bis 20 Gew.-% Lösungsmittel.

Die Erfindung betrifft auch die Verwendung der erfindungsgemässen Dentalwerkstoffe zur Herstellung von dentalen Kompositen, vorzugsweise Komposit-Formkörpern, die insbesondere für die maschinelle Bearbeitung mittels computergestützter Verarbeitungstechniken wie Fräs- und Schleifverfahren geeignet sind und sich vor allem zur Herstellung von dentalen Restaurationsmaterialien wie Inlays, Onlays, Kronen, Brücken oder Verblendmaterialien eignen.

Weiterhin betrifft die Erfindung auch die Verwendung mindestens einer Verbindung der Formel I, und insbesondere mindestens eines Azids der Formel IA und mindestens eines Alkins der Formel IB, wie oben beschrieben zur Herstellung eines Dentalwerkstoffs und insbesondere eines dentalen Kompositmaterials.

Die Erfindung wird im Folgenden anhand von Ausführungsbeispielen näher erläutert.

### Ausführungsbeispiele

### Beispiel 1

### Synthese von 1,2-Bis-(2-azidoethoxy)-ethan

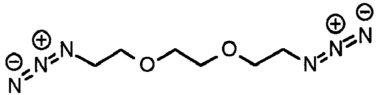

Zu einer Lösung von Triethylenglycol-bis-(4-toluolsulfonat) (10 g, 21,8 mmol) in DMSO (130 ml) wurde Natriumazid (4,25 g, 65,4 mmol, 3 äq.) zugegeben. Die Reaktionsmischung wurde 48 h bei Raumtemperatur gerührt und dann die Reaktion durch Zugabe von Wasser (100 ml) abgebrochen. Das Reaktionsprodukt wurde mit Diethylether (3 x 50 ml) extrahiert und die vereinigten organischen Phasen mit Wasser (3 x 50 ml) gewaschen. Die organische Phase wurde mit wasserfreiem MgSO₄ getrocknet. Nach Entfernen des Lösungsmittels unter vermindertem Druck wurden 4,32 g (98% Ausbeute) des Produkts als gelbes Öl erhalten.
¹H-NMR (300 MHz, CDCl₃): δ = 3,70-3,66 (m, 8H), 3,38 (t, J = 5 Hz, 4H). ¹³C-NMR (75 MHz, CDCl₃): δ = 70,7, 70,1, 50,7.
Anal.: Ber. für C₆H₁₂N₆O₂: C, 36,00%; H, 6,04%; N, 41,98%; gef.: C, 36,25%; H, 5,79%; N, 42,08%.

### Beispiel 2

### Synthese von 1,6-Bis-(prop-2-inoxycarbonylamino)-2,2,4-trimethylhexan

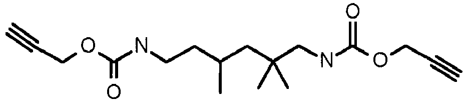

Zu einer Lösung von 2,2,4-Trimethylhexan-1,6-diisocyanat (5 g, 23,8 mmol, Diastereomerenmischung) und Dibutylzinn(II)-dilaurat (6 mg, 9,52 µmol, 0,0004 äq) in Toluol (10 ml) wurde langsam Propargylalkohol (2,67 g, 47,6 mmol, 2 äq.) über einen Zeitraum von 15 min zugetropft und die Reaktionsmischung 48 h bei 60 °C gerührt. Nach Filtration über eine kurze Silica-Säule wurde das Lösungsmittel unter vermindertem Druck entfernt. Es wurden 7,51 g (98% Ausbeute) des Produkts als viskoses gelbes Öl erhalten.
¹H-NMR (300 MHz, CDCl₃): δ = 5, 14-4, 91 (br, 2H, N-H), 4, 65 (br, 4H, C(O)O-CH₂), 3,20-2,88 (m, 4H, NH-CH₂), 2,46 (d, *J* = 1,8 Hz, 2H, C≡CH), 1,70-1,22 (br, 3H), 1,05-0,86 (m, 11H, CH₂, CH₃). ¹³C-NMR (75 MHz, CDCl₃): δ = 155,8, 155,7, 155,4, 129,0, 128,2, 125,3, 78,4, 74,6, 74,5, 52,5, 52,3, 51,4, 48,6, 46,4, 45,9, 41,9, 39,2, 39,1, 37,4, 35,0, 32,9, 29,4, 27,4, 26,1, 25,5, 25,1, 22,3, 21,4, 20,5.
ESI-MS (*m*/*z* (%)): 345,18 (100) [M+ + Na].
Anal.: Ber. für C₁₇H₂₆N₂O₄: C, 63,33%; H, 8,13%; N, 8,69%; gef.: C, 63,07%; H, 7,91%; N, 8,83%.

### Beispiel 3

### Allgemeine Vorschrift für thermische Cu(I)-katalysierte Azid-Alkin-Cyclopolyadditionen

Eine stöchiometrische Mischung von Azid- und Alkin-Verbindungen wurde in ein Gefäss eingewogen und 10 min gerührt. Dann wurde der Kupfer(I)-Katalysator (0,1 bis 5 mol-%) zugegeben und weitere 5 min gerührt, bis sich der Katalysator löste, was typischerweise zur Gelbfärbung der Lösung führte. Dann wurde die Mischung in eine Presshülse aus Metall oder Teflon (0,5-1,0 mm hoch, Innendurchmesser 15 mm) gegeben und mit einer PET-Folie abgedeckt. Die Mischung wurde 24 h bei Raumtemperatur stehen gelassen und anschliessend 24 h auf 32 °C erwärmt, wodurch eine vollständige Aushärtung erreicht wurde. Der Reaktionsverlauf wurde mittels IR-Spektroskopie durch Verfolgung der Abnahme der IR-Absorption der Azidgruppe bei 2100 cm⁻¹ verfolgt.

### Beispiel 4

### Thermische Cu(I)-katalysierte Azid-Alkin-Cyclopolyaddition von 1,2-Bis-(2-azidoethoxy)ethan und 1,6-Bis-(prop-2-in-oxycarbonylamino)2,2,4-trimethylhexan

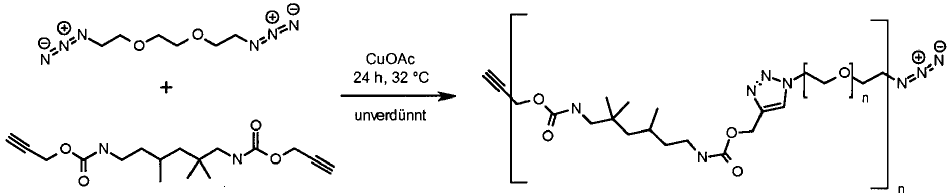

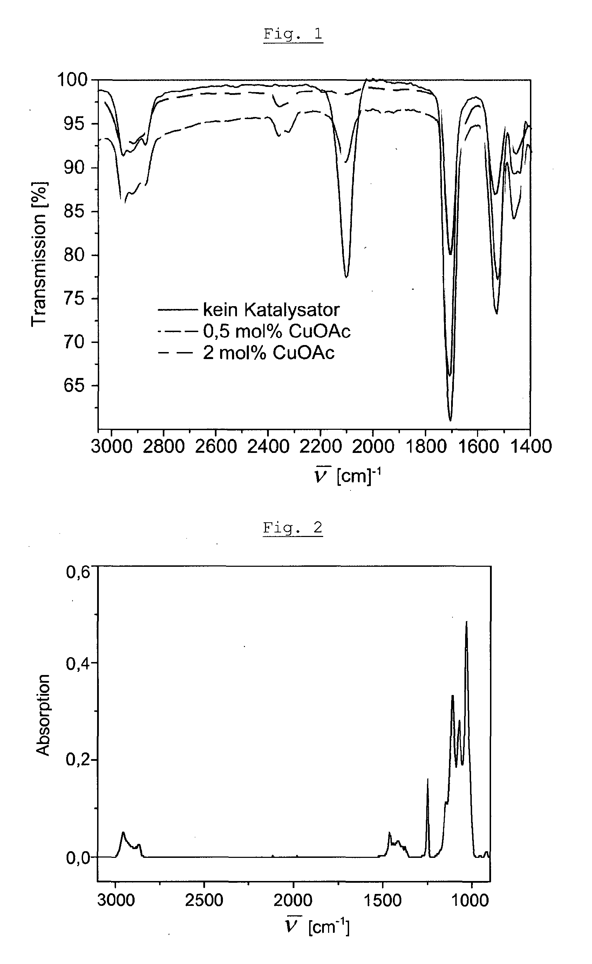

Stöchiometrische Mengen von 1,2-Bis-(2-azidoethoxy)ethan aus Beispiel 1 und 1,6-Bis-(prop-2-inoxycarbonylamino)-2,2,4-trimethylhexan aus Beispiel 2 wurden in Gegenwart von 0,5 mol% bzw. 2 mol% Kupfer(I)-acetat gemäss der vorstehenden allgemeinen Vorschrift umgesetzt. AT-IR-Spektren der erhaltenen Produkte sind in Fig. 1 gezeigt.

### Beispiel 5

### Thermische vernetzende Cu(I)-katalysierte Polycycloaddition einer Mischung von 1,3-Bis-(azidomethyl)benzol und Tripropargylamin

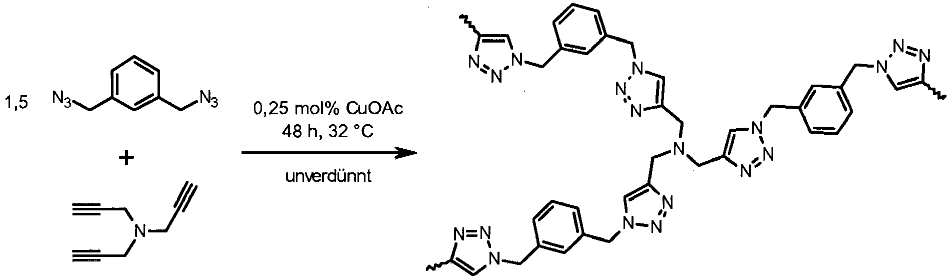

Eine Mischung von Tripropargylamin (Sigma-Aldrich) mit 0,25 mol-% Kupfer(I)-acetat wurde 2 h bei Raumtemperatur gerührt. Danach wurde eine stöchiometrische Menge 1,3-Bis-(azidomethyl)-benzol (hergestellt nach S. T. Abu-Orabi, R. E. Harmon, J. Chem. Eng. Data 31 (1986) 379-380) zugegeben. Zur Härtung wurde die Reaktionsmischung 48 h auf 32 °C erwärmt. Das ATR-IR-Spektrum des erhaltenen vernetzten Polyaddukts ist in Fig. 2 abgebildet und zeigt einen vollständigen Umsatz. Der Elastizitätsmodul dieses Polyaddukts wurde mittels Nanoindentation bestimmt. Dabei handelt es sich um eine örtlich hochauflösende Methode zur mechanischen Charakterisierung von festen Stoffen, die auf der Messung von Kraft und Weg während des elastisch-plastischen Kontakts eines harten Prüfkörpers (Indenter) mit der Probe basiert. Der Wert des mittels Nanoindentation bestimmten Elastizitätsmoduls des vernetzten Polyaddukts betrug 5,5 GPa. Vergleichsmessungen mit einem üblich lichtgehärteten Dentalharz auf Basis einer Mischung von UDMA (90 Gew.-%) und TEGDMA (10 Gew.-%) ergaben ein mittels Nanoindentation bestimmtes Elastizitätsmodul von nur 4,2 GPa.

### Beispiel 6

### Gefülltes Komposit durch thermische vernetzende Cu(I)-katalysierte Polycycloaddition einer Mischung von 1,3-Bis(azidomethyl)benzol und Tripropargylamin

Eine Mischung von Tripropargylamin mit Kupfer(I)-acetat (0,25 mol-%) wurde 2 h bei Raumtemperatur gerührt. Anschliessend wurde eine stöchiometrische Menge 1,3-Bis-(azidomethyl)-benzol zugegeben und in die erhaltene Mischung pyrogene Kieselsäure OX-50 (60 Gew.-% bezogen auf die Gesamtmischung, Primärpartikelgrösse 40 nm, Degussa) eingearbeitet. Zur Härtung wurde die Reaktionsmischung 48 h auf 32 °C erwärmt. Der Wert des mittels Nanoindentation bestimmten Elastizitätsmoduls des erhaltenen gefüllten Komposits betrug 11,7 GPa.

### Beispiel 7

### Photoinduzierte vernetzende Azid-Alkin-Polycycloadditionen einer Mischung von 1,2-Bis-(2-azidoethoxy)-ethan und Tripropargylamin

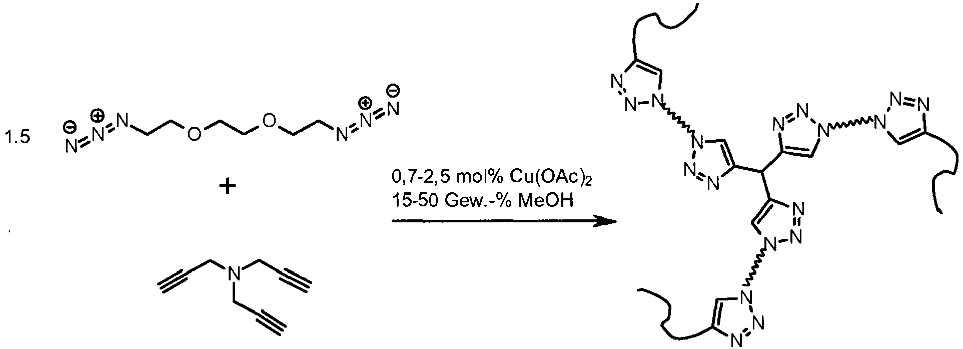

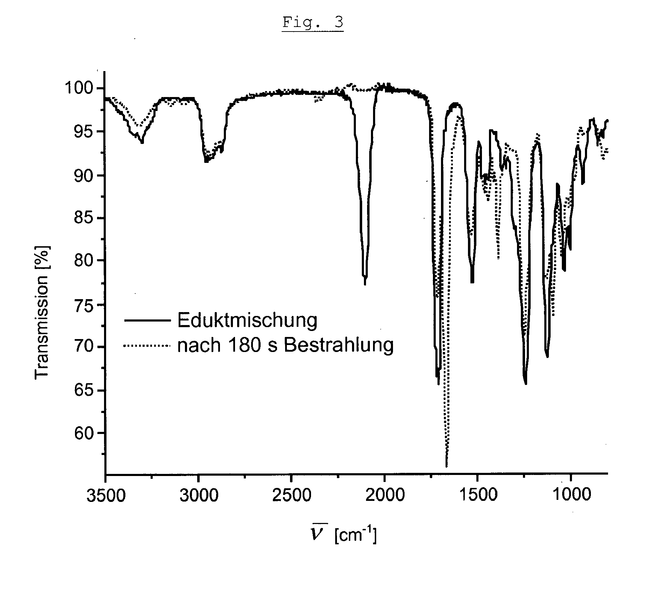

Kupfer(II)-acetat (0,7-2,5 mol-%) wurde innerhalb von 5 min in Tripropargylamin gelöst, wodurch sich die Farbe der Mischung von türkis nach farblos änderte. Nach Zugabe einer stöchiometrischen Menge von 1,2-Bis-(2-azidoethoxy)-ethan und Methanol (15 Gew.-% bezogen auf die Gesamtmischung) wurde die Reaktionsmischung in einem Lichtofen (Dentacolor XS, Kulzer) 180 s lang bestrahlt, wodurch ein farbloses Netzwerkpolymer gebildet wurde. Das ATR-IR-Spektrum des erhaltenen vernetzten Polymers ist in Fig. 3 abgebildet und zeigt einen vollständigen Umsatz.

### Beispiel 8

### Thermische Alkin-Azid-Cycloaddition und Photopolymerisation eines Hybridsystems auf Basis einer Mischung von 1,3-Bis-(azidomethyl)-benzol, Tripropargylamin und N,N'-Diethyl-l,3-bis-(acrylamido)-propan

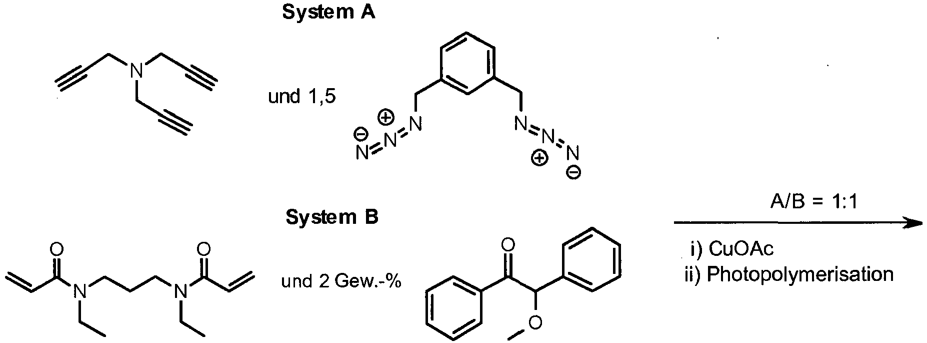

Zur Herstellung von System A wurde eine Mischung von Tripropargylamin (1 mol) und Kupfer(I)-acetat (0,16 mol%) mind. 2 h gerührt, bis eine deutliche Gelbfärbung zu erkennen war, und danach wurde zu der 1,3-Bis-(azidomethyl)-benzol (1,5 mol) zugegeben. Zur Herstellung von System B wurde der Photoinitiator Benzoylmethylether (2 Gew.-%) innerhalb von 30 min in N,N'-Diethyl-1,3-bis-(acrylamido)-propan gelöst. Anschliessend wurden gleiche Gewichtsteile der Systeme A und B gemischt (A/B = 1:1). Die erhaltene Mischung wurde zur Härtung 24 h bei Raumtemperatur stehen gelassen, dann in einem Lichtofen (Dentacolor XS, Kulzer) 540 s lang bestrahlt und schliesslich weitere 24 h auf 32 °C erwärmt. Das ATR-IR-Spektrum des erhaltenen vernetzten Polymers zeigte einen vollständigen Umsatz. Der Wert des mittels Nanoindentation bestimmten Elastizitätsmoduls betrug 5,1 GPa.

## Patentansprüche

1. Dentalwerkstoff, der mindestens eine Verbindung der Formel I enthält
A-[X-Q-(Y-CG)ₙ]ₘ Formel I,
in der
A für -O-, -N=, -NR³-, =N-N=, -NR³-N=, =N-NR³-, -NR³-NR³-oder einen m-wertigen Alkylen-Rest, Cycloalkylen-Rest, Heterocycloalkylen-Rest, Arylen-Rest, Heteroarylen-Rest, gemischten Alkylen-Cyclyl-Rest oder Cyclyl-O-Cyclyl-Rest steht, wobei
Alkylen jeweils unabhängig für einen linearen oder verzweigten aliphatischen C₁-C₅₀-Rest steht, der durch ein oder mehrere -O-, -S-, -NR³-, -CO-O-, -O-CO-, -O-CO-O-, -CO-NR³-, -NR³-CO-, -O-CO-NR³-, -NR³-CO-O- oder -NR³-CO-NR³- unterbrochen sein kann,
Cycloalkylen jeweils unabhängig für einen cycloaliphatischen C₃-C₁₈-Rest steht,
Heterocycloalkylen jeweils unabhängig für einen heterocycloaliphatischen C₃-C₁₈-Rest steht,
Arylen jeweils unabhängig für einen aromatischen C₆-C₁₈-Rest steht,
Heteroarylen jeweils unabhängig für einen heteroaromatischen C₃-C₁₈-Rest steht,
Cyclyl jeweils unabhängig für Cycloalkylen, Heterocycloalkylen, Arylen oder Heteroarylen steht und
die genannten Reste jeweils unabhängig einen oder mehrere Substituenten, insbesondere ausgewählt aus C₁-C₅-Alkyl, OH, OCH₃ und OCOCH₃, tragen können,
CG jeweils unabhängig für eine Azidgruppe N₃ oder eine Alkingruppe ausgewählt aus der Gruppe bestehend aus -CR¹R²-C≡CH, 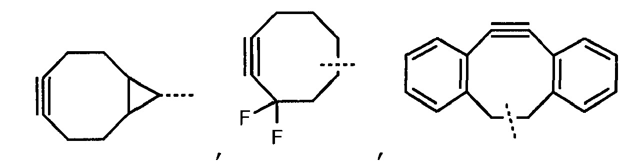 und 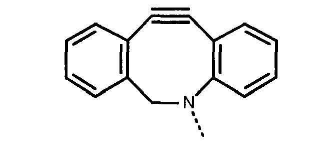 steht,
mit der Massgabe, dass das Dentalwerkstoff mindestens eine Verbindung der Formel I, die eine Azidgruppe aufweist, und mindestens eine Verbindung der Formel I enthält, die eine Alkingruppe aufweist,
Q jeweils unabhängig entfällt oder für einen (n+1)-wertigen linearen oder verzweigten aliphatischen C₁-C₂₀-Alkylen-Rest, der durch ein oder mehrere -O- oder -S- unterbrochen sein kann, oder einen Phenylenrest steht, wobei die genannten Reste jeweils unabhängig einen oder mehrere Substituenten, insbesondere ausgewählt aus CH₃, C₂H₅, OH, OCH₃ und OCOCH₃, tragen können,
R¹, R² und R³ jeweils unabhängig für H oder einen C₁-C₁₀-Alkylrest stehen,
X und Y jeweils unabhängig entfallen oder für -O-, -S-, -NR³-, -CO-, -CO-O-, -O-CO-, -O-CO-O-, -CO-NR³-, -NR³-CO-, -O-CO-NR³-, -NR³-CO-O- oder -NR³-CO-NR³- stehen,
m jeweils unabhängig die Werte 2 bis 6 annehmen kann und
n jeweils unabhängig die Werte 1 bis 4 annehmen kann.

2. Dentalwerkstoff nach Anspruch 1, bei dem jeweils unabhängig voneinander
A für -N= oder einen m-wertigen Alkylen-Rest, Cycloalkylen-Rest, Heterocycloalkylen-Rest, Arylen-Rest, Heteroarylen-Rest, gemischten Alkylen-Cyclyl-Rest oder Cyclyl-O-Cyclyl-Rest steht, wobei
Alkylen jeweils unabhängig für einen linearen oder verzweigten aliphatischen C₁-C₂₀-Rest steht, der durch ein oder mehrere -O-, -S-, -CO-O-, -O-CO- oder -O-CO-O- unterbrochen sein kann,
Cycloalkylen jeweils unabhängig für einen cycloaliphatischen C₃-C₁₂-Rest steht,
Heterocycloalkylen jeweils unabhängig für einen heterocycloaliphatischen C₃-C₁₂-Rest steht,
Arylen jeweils unabhängig für einen aromatischen C₆-C₁₂-Rest steht,
Heteroarylen jeweils unabhängig für einen heteroaromatischen C₃-C₁₂-Rest steht,
Cyclyl jeweils unabhängig für Cycloalkylen, Heterocycloalkylen, Arylen oder Heteroarylen steht und
die genannten Reste jeweils unabhängig einen oder mehrere Substituenten, insbesondere ausgewählt aus C₁-C₃-Alkyl, OCH₃ und OCOCH₃, tragen können,
CG jeweils unabhängig für eine Azidgruppe N₃ oder eine Alkingruppe ausgewählt aus der Gruppe bestehend aus -CR¹R²-C≡CH, 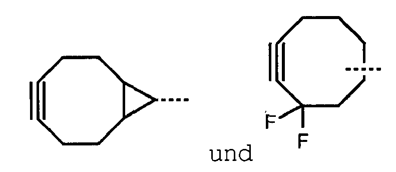 steht,
Q jeweils unabhängig entfällt oder für einen (n+1)-wertigen linearen oder verzweigten aliphatischen C₁-C₁₀-Alkylen-Rest, der durch ein oder mehrere -O- oder -S-unterbrochen sein kann, oder einen Phenylenrest steht, wobei die genannten Reste jeweils unabhängig einen oder mehrere Substituenten, insbesondere ausgewählt aus CH₃, C₂H₅, OCH₃ und OCOCH₃, tragen können,
R¹, R² und R³ jeweils unabhängig für H oder einen C₁-C₅-Alkylrest und insbesondere einen C₁-C₃-Alkylrest stehen,
X und Y jeweils unabhängig entfallen oder für -O-, -S-, -CO-O-, -O-CO-, -O-CO-O-, -CO-NR³-, -NR³-CO-, -O-CO-NR³-, -NR³-CO-O-oder -NR³-CO-NR³- stehen,
m jeweils unabhängig die Werte 2, 3 oder 4 annehmen kann,
n jeweils unabhängig die Werte 1, 2 oder 3 und insbesondere 1 oder 2 annehmen kann und am meisten bevorzugt 1 ist.

3. Dentalwerkstoff nach Anspruch 1 oder 2, der mindestens ein Azid der Formel IA
A-[X-Q-(Y-CG)ₙ]ₘ Formel IA
in der
A für -O-, -N=, -NR³-, =N-N=, -NR³-N=, =N-NR³-, -NR³-NR³-oder einen m-wertigen Alkylen-Rest, Cycloalkylen-Rest, Heterocycloalkylen-Rest, Arylen-Rest, Heteroarylen-Rest, gemischten Alkylen-Cyclyl-Rest oder Cyclyl-O-Cyclyl-Rest steht, wobei
Alkylen jeweils unabhängig für einen linearen oder verzweigten aliphatischen C₁-C₅₀-Rest steht, der durch ein oder mehrere -O-, -S-, -NR³-, -CO-O-, -O-CO-, -O-CO-O-, -CO-NR³-, -NR³-CO-, -O-CO-NR³-, -NR³-CO-O- oder -NR³-CO-NR³- unterbrochen sein kann,
Cycloalkylen jeweils unabhängig für einen cycloaliphatischen C₃-C₁₈-Rest steht,
Heterocycloalkylen jeweils unabhängig für einen heterocycloaliphatischen C₃-C₁₈-Rest steht,
Arylen jeweils unabhängig für einen aromatischen C₆-C₁₈-Rest steht,
Heteroarylen jeweils unabhängig für einen heteroaromatischen C₃-C₁₈-Rest steht,
Cyclyl jeweils unabhängig für Cycloalkylen, Heterocycloalkylen, Arylen oder Heteroarylen steht und
die genannten Reste jeweils unabhängig einen oder mehrere Substituenten, insbesondere ausgewählt aus C₁-C₅-Alkyl, OH, OCH₃ und OCOCH₃, tragen können,
CG jeweils unabhängig für eine Azidgruppe N₃ steht,
Q jeweils unabhängig entfällt oder für einen (n+1)-wertigen linearen oder verzweigten aliphatischen C₁-C₂₀-Alkylen-Rest, der durch ein oder mehrere -O- oder -S- unterbrochen sein kann, oder einen Phenylenrest steht, wobei die genannten Reste jeweils unabhängig einen oder mehrere Substituenten, insbesondere ausgewählt aus CH₃, C₂H₅, OH, OCH₃ und OCOCH₃, tragen können,
R¹, R² und R³ jeweils unabhängig für H oder einen C₁-C₁₀-Alkylrest stehen,
X und Y jeweils unabhängig entfallen oder für -O-, -S-, -NR³-, -CO-, -CO-O-, -O-CO-, -O-CO-O-, -CO-NR³-, -NR³-CO-, -O-CO-NR³-, -NR³-CO-O- oder -NR³-CO-NR³- stehen,
m jeweils unabhängig die Werte 2 bis 6 annehmen kann und
n jeweils unabhängig die Werte 1 bis 4 annehmen kann, und
mindestens ein Alkin der Formel IB enthält
A- [X-Q- (Y-CG) n] m Formel IB
in der
A für -O-, -N=, -NR³-, =N-N=, -NR³-N=, =N-NR³-, -NR³-NR³-oder einen m-wertigen Alkylen-Rest, Cycloalkylen-Rest, Heterocycloalkylen-Rest, Arylen-Rest, Heteroarylen-Rest, gemischten Alkylen-Cyclyl-Rest oder Cyclyl-O-Cyclyl-Rest steht, wobei
Alkylen jeweils unabhängig für einen linearen oder verzweigten aliphatischen C₁-C₅₀-Rest steht, der durch ein oder mehrere -O-, -S-, -NR³-, -CO-O-, -O-CO-, -O-CO-O-, -CO-NR³-, -NR³-CO-, -O-CO-NR³-, -NR³-CO-O- oder -NR³-CO-NR³- unterbrochen sein kann,
Cycloalkylen jeweils unabhängig für einen cycloaliphatischen C₃-C₁₈-Rest steht,
Heterocycloalkylen jeweils unabhängig für einen heterocycloaliphatischen C₃-C₁₈-Rest steht,
Arylen jeweils unabhängig für einen aromatischen C₆-C₁₈-Rest steht,
Heteroarylen jeweils unabhängig für einen heteroaromatischen C₃-C₁₈-Rest steht,
Cyclyl jeweils unabhängig für Cycloalkylen, Heterocycloalkylen, Arylen oder Heteroarylen steht und
die genannten Reste jeweils unabhängig einen oder mehrere Substituenten, insbesondere ausgewählt aus C₁-C₅-Alkyl, OH, OCH₃ und OCOCH₃, tragen können,
CG jeweils unabhängig für eine Alkingruppe ausgewählt aus der Gruppe bestehend aus -CR¹R²-C≡CH, 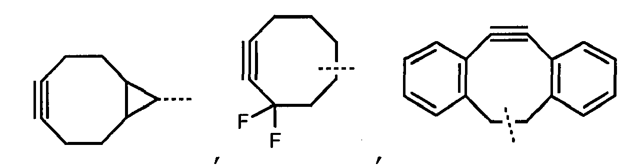 und 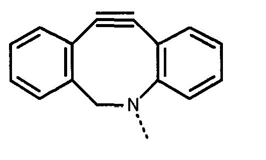 steht,
Q jeweils unabhängig entfällt oder für einen (n+1)-wertigen linearen oder verzweigten aliphatischen C₁-C₂₀-Alkylen-Rest, der durch ein oder mehrere -O- oder -S- unterbrochen sein kann, oder einen Phenylenrest steht, wobei die genannten Reste jeweils unabhängig einen oder mehrere Substituenten, insbesondere ausgewählt aus CH₃, C₂H₅, OH, OCH₃ und OCOCH₃, tragen können,
R¹, R² und R³ jeweils unabhängig für H oder einen C₁-C₁₀-Alkylrest stehen,
X und Y jeweils unabhängig entfallen oder für -O-, -S-, -NR³-, -CO-, -CO-O-, -O-CO-, -O-CO-O-, -CO-NR³-, -NR³-CO-, -O-CO-NR³-, -NR³-CO-O- oder -NR³-CO-NR³- stehen,
m jeweils unabhängig die Werte 2 bis 6 annehmen kann und
n jeweils unabhängig die Werte 1 bis 4 annehmen kann.

4. Dentalwerkstoff nach einem der Ansprüche 1 bis 3, in dem die Verbindung der Formel I eine mittlere Funktionalität von > 2,2 und insbesondere > 2,5 aufweist.

5. Dentalwerkstoff nach einem der Ansprüche 1 bis 4, der einen Katalysator für die Azid-Alkin-Cycloaddition enthält.

6. Dentalwerkstoff nach Anspruch 5, der einen Katalysator ausgewählt aus der Gruppe bestehend aus Kupfer-, Ruthenium-, Nickel-, Palladium- und Platin-Verbindungen und -Komplexen enthält.

7. Dentalwerkstoff nach Anspruch 5 oder 6, der als Katalysator eine Kupfer(II)-Verbindung und ein lichtinduzierbares Reduktionsmittel enthält.

8. Dentalwerkstoff nach Anspruch 6 oder 7, der eine Kupfer(II)-Verbindung ausgewählt aus CuSO₄, CuCl₂, CuBr₂, Cu(OH)₂, Cu(OAc)₂, CuO, Cu(ClO₄)₂ und Cu(NO₃)₂ sowie deren Hydraten und THF-Addukten enthält.

9. Dentalwerkstoff nach Anspruch 7 oder 8, der als lichtinduzierbares Reduktionsmittel einen Photoinitiator ausgewählt aus der Gruppe bestehend aus Norrish-Typ-I-Photoinitiatoren, Titanocenen und α-Diketonen enthält.

10. Dentalwerkstoff nach einem der Ansprüche 1 bis 9, der ein oder mehrere radikalisch polymerisierbare Monomere enthält.

11. Dentalwerkstoff nach Anspruch 10, der
Methyl-, Ethyl-, Hydroxyethyl-, Butyl-, Benzyl-, Tetrahydrofurfuryl- oder Isobornyl(meth)acrylat, p-Cumyl-phenoxyethylenglycolmethacrylat (CMP-1E), Bisphenol-A-di(meth)acrylat, Bis-GMA, ethoxy- oder propoxyliertes Bisphenol-A-Dimethacrylat, UDMA, Di-, Tri- oder Tetraethylenglycoldi(meth)acrylat, Trimethylolpropantri(meth)acrylat, Pentaerythrittetra(meth)acrylat, Glycerindi- oder -tri(meth)acrylat, 1,4-Butandioldi(meth)acrylat, 1,10-Decandioldi(meth)acrylat (D₃MA), 1,12-Dodecandioldi-(meth)acrylat,
und/oder
ein oder mehrere N-mono- oder -disubstitiuierte Acrylamide, N-Ethylacrylamid, N,N-Dimethacrylamid, N-(2-Hydroxyethyl)-acryl-amid, N-Methyl-N-(2-hydroxyethyl)acrylamid, ein oder mehrere N-monosubstituierte Methacrylamide, N-Ethylmethacrylamid, N-(2-Hydroxyethyl)methacrylamid, N-Vinylpyrrolidon, ein oder mehrere vernetzende Allylether,
und/oder
ein oder mehrere vernetzende Pyrrolidone, 1,6-Bis(3-vinyl-2-pyrrolidonyl)-hexan, ein oder mehrere vernetzende Bisacrylamide, Methylen- oder Ethylenbisacrylamid, ein oder mehrere vernetzende Bis(meth)acrylamide, N,N'-Diethyl-1,3-bis(acrylamido)-propan, 1,3-Bis(methacrylamido)-propan, 1,4-Bis(acrylamido)-butan, 1,4-Bis(acryloyl)-piperazin,
oder eine Mischung davon enthält.

12. Dentalwerkstoff nach einem der Ansprüche 1 bis 11, der organischen und/oder anorganischen Füllstoff enthält.

13. Dentalwerkstoff nach einem der Ansprüche 1 bis 12, der
a) 0,1 bis 80 Gew.-%, insbesondere 1 bis 60 Gew.-%, bevorzugt 5 bis 50 Gew.-% und besonders bevorzugt 10 bis 40 Gew.-% mindestens einer Verbindung der Formel I und vorzugsweise einer Mischung mindestens eines Azids der Formel IA und mindestens eines Alkins der Formel IB,
b) 0,01 bis 10 Gew.-%, bevorzugt 0,1 bis 3 Gew.-% und besonders bevorzugt 0,2 bis 2 Gew.-% Katalysator,
c) 0 bis 80 Gew.-%, bevorzugt 1 bis 60 Gew.-% und besonders bevorzugt 5 bis 50 Gew.-% Co-Monomer,
d) 0 bis 90 Gew.-%, bevorzugt 10 bis 80 Gew.-% und besonders bevorzugt 20 bis 70 Gew.-% Füllstoff und
e) 0 bis 70 Gew.-%, bevorzugt 1 bis 50 Gew.-% und besonders bevorzugt 5 bis 20 Gew.-% Lösungsmittel.

14. Verwendung eines Dentalwerkstoffs gemäss einem der Ansprüche 1 bis 13 zur Herstellung von dentalen Kompositen oder dentalen Restaurationsmaterialien und insbesondere zur Herstellung von Inlays, Onlays, Kronen, Brücken oder Verblendmaterialien.

15. Verwendung mindestens einer Verbindung der Formel I wie in einem der Ansprüche 1 bis 4 definiert, und insbesondere mindestens eines Azids der Formel IA und mindestens eines Alkins der Formel IB wie in Anspruch 3 oder 4 definiert, zur Herstellung eines Dentalwerkstoffs und insbesondere eines dentalen Kompositmaterials.

## Claims

1. Dental material which comprises at least one compound of Formula I
A-[X-Q-(Y-CG)ₙ]ₘ Formula I,
in which
A stands for -O-, -N=, -NR³-, =N-N=, -NR³-N=, =N-NR³-, -NR³-NR³- or an m-valent alkylene residue, cycloalkylene residue, heterocycloalkylene residue, arylene residue, heteroarylene residue, mixed alkylene-cyclyl residue or cyclyl-O-cyclyl residue, wherein
alkylene, in each case, stands independently for a linear or branched aliphatic C₁-C₅₀ residue that can be interrupted by one or more -O-, -S-, -NR³-, -CO-O-, -O-CO-, -O-CO-O-, -CO-NR³-, - NR³-CO-, -O-CO-NR³-, -NR³-CO-O- or -NR³-CO-NR³-,
cycloalkylene, in each case, stands independently for a cycloaliphatic C₃-C₁₈ residue,
heterocycloalkylene, in each case, stands independently for a heterocycloaliphatic C₃-C₁₈ residue,
arylene, in each case, stands independently for an aromatic C₆-C₁₈ residue,
heteroarylene, in each case, stands independently for a heteroaromatic C₃-C₁₈ residue,
cyclyl, in each case, stands independently for cycloalkylene, heterocycloalkylene, arylene or heteroarylene and
the indicated residues, in each case, independently can carry one or more substituents, in particular selected from C₁-C₅ alkyl, OH, OCH₃ and OCOCH₃,
CG in each case stands independently for an azide group N₃ or an alkyne group selected from the group consisting of -CR¹R²-C≡CH, 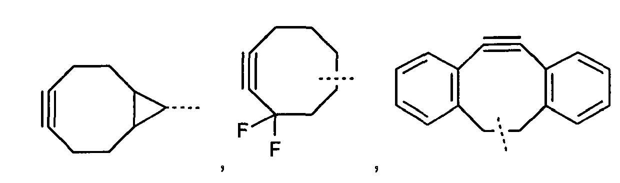 and 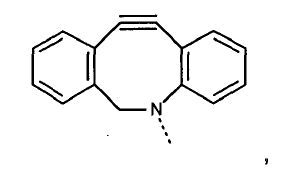 with the proviso that the dental material comprises at least one compound of Formula I which comprises an azide group, and at least one compound of Formula I which comprises an alkyne group,
Q in each case independently is missing or stands for an (n+1)-valent linear or branched aliphatic C₁-C₂₀ alkylene residue that can be interrupted by one or more -O- or -S-, or stands for a phenylene residue, wherein the indicated residues in each case independently can carry one or more substituents, in particular selected from CH₃, C₂H₅, OH, OCH₃ and OCOCH₃,
R¹, R² and R³, in each case, independently stand for H or a C₁-C₁₀ alkyl residue,
X and Y, in each case, independently are missing or stand for -O-, -S-, - NR³-, -CO-, -CO-O-, -O-CO-, -O-CO-O-, -CO-NR³-, -NR³-CO-, -O-CO-NR³-, -NR³-CO-O- or -NR³-CO-NR³-,
m in each case independently can assume the values 2 to 6 and
n in each case independently can assume the values 1 to 4.

2. Dental material according to claim 1, wherein in each case, independently of each other
A stands for -N= or an m-valent alkylene residue, cycloalkylene residue, heterocycloalkylene residue, arylene residue, heteroarylene residue, mixed alkylene-cyclyl residue or cyclyl-O-cyclyl residue, wherein
alkylene, in each case, independently stands for a linear or branched aliphatic C₁-C₂₀ residue that can be interrupted by one or more -O-, -S-, -CO-O-, -O-CO- or -O-CO-O-,
cycloalkylene, in each case, independently stands for a cycloaliphatic C₃-C₁₂ residue,
heterocycloalkylene, in each case, independently stands for a heterocycloaliphatic C₃-C₁₂ residue,
arylene, in each case, independently stands for an aromatic C₆-C₁₂ residue,
heteroarylene, in each case, independently stands for a heteroaromatic C₃-C₁₂ residue,
cyclyl, in each case, independently stands for cycloalkylene, heterocycloalkylene, arylene or heteroarylene and
the indicated residues in each case independently can carry one or more substituents, in particular selected from C₁-C₃ alkyl, OCH₃ and OCOCH₃,
CG in each case independently stands for an azide group N₃ or an alkyne group selected from the group consisting of -CR¹R²-C≡CH, 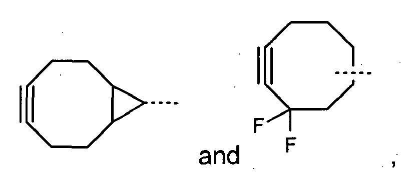
Q in each case independently is missing or stands for an (n+1)-valent linear or branched aliphatic C₁-C₁₀ alkylene residue that can be interrupted by one or more -O- or -S-, or stands for a phenylene residue, wherein the indicated residues in each case independently can carry one or more substituents, in particular selected from CH₃, C₂H₅, OCH₃ and OCOCH₃,
R¹, R² and R³, in each case, independently stand for H or a C₁-C₅ alkyl residue and in particular a C₁-C₃ alkyl residue,
X and Y in each case independently are missing or stand for -O-, -S-, - CO-O-, -O-CO-, -O-CO-O-, -CO-NR³-, -NR³-CO-, -O-CO-NR³-, - NR³-CO-O- or -NR³-CO-NR³-,
m in each case independently can assume the values 2, 3 or 4,
n in each case independently can assume the values 1, 2 or 3, and in particular 1 or 2, and most preferably is 1.

3. Dental material according to claim 1 or 2, which comprises at least one azide of Formula IA
A-[X-Q-(Y-CG)ₙ]ₘ Formula IA
in which
A stands for -O-, -N=, -NR³-, =N-N=, -NR³-N=, =N-NR³-, -NR³-NR³-or an m-valent alkylene residue, cycloalkylene residue, heterocycloalkylene residue, arylene residue, heteroarylene residue, mixed alkylene-cyclyl residue or cyclyl-O-cyclyl residue, wherein
alkylene, in each case, independently stands for a linear or branched aliphatic C₁-C₅₀ residue that can be interrupted by one or more -O-, -S-, -NR³-, -CO-O-, -O-CO-, -O-CO-O-, -CO-NR³-, - NR³-CO-, -O-CO-NR³-, -NR³-CO-O- or -NR³-CO-NR³-,
cycloalkylene, in each case, independently stands for a cycloaliphatic C₃-C₁₈ residue,
heterocycloalkylene, in each case, independently stands for a heterocycloaliphatic C₃-C₁₈ residue,
arylene, in each case, independently stands for an aromatic C₆-C₁₈ residue,
heteroarylene, in each case, independently stands for a heteroaromatic C₃-C₁₈ residue,
cyclyl, in each case, independently stands for cycloalkylene, heterocycloalkylene, arylene or heteroarylene and
the indicated residues in each case independently can carry one or more substituents, in particular selected from C₁-C₅ alkyl, OH, OCH₃ and OCOCH₃,
CG in each case independently stands for an azide group N₃,
Q in each case independently is missing or represents an (n+1)-valent linear or branched aliphatic C₁-C₂₀ alkylene residue that can be interrupted by one or more -O- or -S-, or stands for a phenylene residue, wherein the indicated residues in each case independently can carry one or more substituents, in particular selected from CH₃, C₂H₅, OH, OCH₃ and OCOCH₃,
R¹, R² and R³, in each case, independently stand for H or a C₁-C₁₀ alkyl residue,
X and Y in each case independently are missing or stand for -O-, -S-, - NR³-, -CO-, -CO-O-, -O-CO-, -O-CO-O-, -CO-NR³-, -NR³-CO-, - O-CO-NR³-, -NR³-CO-O- or-NR³-CO-NR³-,
m in each case independently can assume the values 2 to 6 and
n in each case independently can assume the values 1 to 4, and
at least one alkyne of Formula IB
A-[X-Q-(Y-CG)ₙ]ₘ Formula IB
in which
A stands for -O-, -N=, -NR³-, =N-N=, -NR³-N=, =N-NR³-, -NR³-NR³- or an m-valent alkylene residue, cycloalkylene residue, heterocycloalkylene residue, arylene residue, heteroarylene residue, mixed alkylene-cyclyl residue or cyclyl-O-cyclyl residue, wherein alkylene, in each case, independently stands for a linear or branched aliphatic C₁-C₅₀ residue that can be interrupted by one or more -O-, - S-, -NR³-, -CO-O-, -O-CO-, -O-CO-O-, -CO-NR³-, -NR³-CO-, -O-CO-NR³-, -NR³-CO-O- or -NR³-CO-NR³-,
cycloalkylene, in each case, independently stands for a cycloaliphatic C₃-C₁₈ residue,
heterocycloalkylene, in each case, independently stands for a heterocycloaliphatic C₃-C₁₈ residue,
arylene, in each case, independently stands for an aromatic C₆-C₁₈ residue,
heteroarylene, in each case, independently stands for a heteroaromatic C₃-C₁₈ residue,
cyclyl, in each case, independently stands for cycloalkylene, heterocycloalkylene, arylene or heteroarylene and
the indicated residues in each case independently can carry one or more substituents, in particular selected from C₁-C₅ alkyl, OH, OCH₃ and OCOCH₃,
CG in each case independently stands for an alkyne group selected from the group consisting of -CR¹R²-C≡CH, 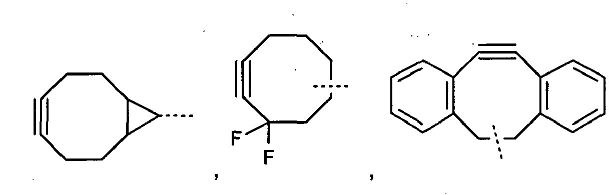 and 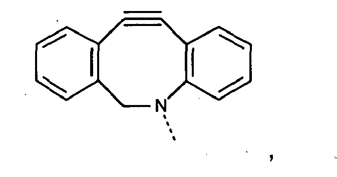
Q in each case independently is missing or stands for an (n+1)-valent linear or branched aliphatic C₁-C₂₀ alkylene residue that can be interrupted by one or more -O- or -S-, or stands for a phenylene residue, wherein the indicated residues in each case independently can carry one or more substituents, in particular selected from CH₃, C₂H₅, OH, OCH₃ and OCOCH₃,
R¹, R² and R³, in each case, independently stands for a C₁-C₁₀ alkyl residue,
X and Y in each case independently are missing or stand for -O-, -S-, -NR³-, -CO-, -CO-O-, -O-CO-, -O-CO-O-, -CO-NR³-, -NR³-CO-, -O-CO-NR³-, -NR³-CO-O- or -NR³-CO-NR³-,
m in each case independently can assume the values 2 to 6 and
n in each case independently can assume the values 1 to 4.

4. Dental material according to one of claims 1 to 3, wherein the compound of Formula I has an average functionality of > 2.2 and in particular > 2.5.

5. Dental material according to one of claims 1 to 4, which comprises a catalyst for the azide-alkyne cycloaddition.

6. Dental material according to claim 5, which comprises a catalyst selected from the group consisting of compounds and complexes of copper, ruthenium, nickel, palladium and platinum.

7. Dental material according to claim 5 or 6, which comprises a copper(II) compound as the catalyst and a light-inducible reducing agent.

8. Dental material according to claim 6 or 7, which comprises a copper(II) compound selected from CuSO4, CuCl2, CuBr2, Cu(OH)2, Cu(OAc)2, CuO, Cu(ClO4)2 and Cu(N03)2 as well as their hydrates and THF adducts.

9. Dental material according to claim 7 or 8, which comprises as the light-inducible reducing agent a photoinitiator selected from the group consisting of Norrish type I photoinitiators, titanocenes and α-diketones.

10. Dental material according to any one of claims 1 to 9, which comprises one or more radically polymerisable monomers.

11. Dental material according to claim 10, which comprises
methyl, ethyl, hydroxyethyl, butyl, benzyl, tetrahydrofurfuryl or isobornyl (meth)acrylate esters, p-cumyl-phenoxyethylene glycol methacrylate (CMP-1E), bisphenol-A di(meth)acrylate, Bis-GMA, ethoxylated or propoxylated bisphenol-A dimethacrylate, UDMA, di, tri or tetraethylene glycol di(meth)acrylate, trimethylolpropane tri(meth)acrylate, pentaerythritol tetra(meth)acrylate, glycerol di or tri(meth)acrylate, 1,4-butanediol di(meth)acrylate, 1,10-decanediol di(meth)acrylate (D₃MA), 1,12-dodecanediol di(meth)acrylate,
and/or
one or more N-mono or -disubstituted acrylamides, N-ethylacrylamide, N,N-dimethacrylamide, N-(2-hydroxyethyl)acrylamide, N-methyl-N-(2-hydroxyethyl)acrylamide, one or more N-monosubstituted methacrylamides, N-ethylmethacrylamide, N-(2-hydroxyethyl)methacrylamide, N-vinylpyrrolidone, one or more cross-linking allyl ethers,
and/or
one or more cross-linking pyrrolidones, 1,6-bis(3-vinyl-2-pyrrolidonyl)-hexane, one or more cross-linking bisacrylamides, methylene or ethylene bisacrylamide, one or more cross-linking bis(meth)acrylamides, N,N'-diethyl-1,3-bis(acrylamido)-propane, 1,3-bis(methacrylamido)-propane, 1,4-bis(acrylamido)-butane, 1,4-bis(acryloyl)-piperazine,
or a mixture thereof.

12. Dental material according to one of claims 1 to 11, which comprises organic and/or inorganic filler.

13. Dental material according to one of claims 1 to 12, which comprises
a) 0.1 to 80 wt %, in particular 1 to 60 wt %, preferably 5 to 50 wt % and particularly preferably 10 to 40 wt % of at least one compound of Formula I and preferably of a mixture of at least one azide of Formula IA and at least one alkyne of Formula IB,
b) 0.01 to 10 wt %, preferably 0.1 to 3 wt % and particularly preferably 0.2 to 2 wt % catalyst,
c) 0 to 80 wt %, preferably 1 to 60 wt % and particularly preferably 5 to 50 wt % co-monomer,
d) 0 to 90 wt %, preferably 10 to 80 wt % and particularly preferably 20 to 70 wt % filler and
e) 0 to 70 wt %, preferably 1 to 50 wt % and particularly preferably 5 to 20 wt % solvent.

14. Use of a dental material according to one of claims 1 to 13 for preparing dental composites or dental restoration materials, and in particular for preparing inlays, onlays, crowns, bridges or veneering materials.

15. Use of at least one compound of Formula I as defined in one of claims 1 to 4, and in particular of at least one azide of Formula IA and at least one alkyne of Formula IB as defined in claim 3 or 4, for preparing a dental material and in particular a dental composite material.

## Revendications

1. Matériau dentaire qui contient au moins un composé de formule I :
A-[X-Q-(Y-CG)ₙ]ₘ Formule I
dans laquelle
- A représente un chaînon ou raccord symbolisé par -O-, -N=, -NR³-, =N-N=, -NR³-N=, =N-NR³- ou -NR³-NR³-, ou un groupe de valence m et de type alkylène, cycloalkylène, hétérocycloalkylène, arylène, hétéroarylène, mixte alkylène-cyclyle ou cyclyl-oxy-cyclyle, étant entendu que :
le terme « alkylène » désigne, indépendamment dans chaque cas, un groupe aliphatique en C₁-C₅₀, linéaire ou ramifié, qui peut être interrompu par un ou plusieurs chaînon(s) ou raccord(s) symbolisé(s) par -O-, -S-, -NR³-, -CO-O-, -O-CO-, -O-CO-O-, -CO-NR³-, -NR³-CO-, -O-CO-NR³-, -NR³-CO-O-, ou -NR³-CO-NR³,
le terme « cycloalkylène » désigne, indépendamment dans chaque cas, un groupe cycloaliphatique en C₃-C₁₈,
le terme « hétérocycloalkylène » désigne, indépendamment dans chaque cas, un groupe hétérocycloaliphatique en C₃-C₁₈,
le terme « arylène » désigne, indépendamment dans chaque cas, un groupe aromatique en C₆-C₁₈,
le terme « hétéroarylène » désigne, indépendamment dans chaque cas, un groupe hétéroaromatique en C₃-C₁₈,
le terme « cyclyle » désigne, indépendamment dans chaque cas, un groupe de type cycloalkylène, hétérocycloalkylène, arylène ou hétéroarylène,
et les groupes indiqués ci-dessus peuvent porter chacun, indépendamment, un ou plusieurs substituant(s), choisi(s) en particulier parmi les groupes alkyle en C₁-C₅ et les groupes symbolisés par OH, OCH₃ et OCOCH₃ ;
- CG représente, indépendamment en chaque occurrence, un groupe azoture N₃ ou un groupe alcyne choisi dans l'ensemble formé par les groupes de formule -CR¹R²-C≡CH, 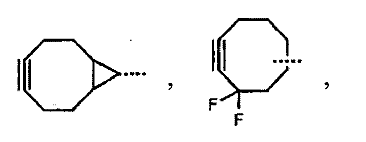 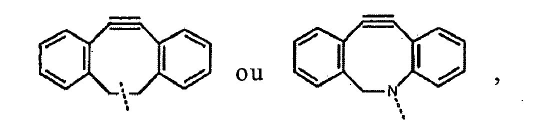 sous réserve que le matériau dentaire contienne au moins un composé de formule I comportant un groupe azoture, ainsi qu'au moins un composé de formule I comportant un groupe alcyne ;
- Q, indépendamment en chaque occurrence, ne représente rien ou représente un groupe de valence n+1 et de type alkylène en C₁-C₂₀, aliphatique linéaire ou ramifié, qui peut être interrompu par un ou plusieurs chaînon(s) symbolisé(s) par -O- ou -S-, ou un groupe de type phénylène, étant entendu que les groupes indiqués ci-dessus peuvent porter chacun, indépendamment, un ou plusieurs substituant(s), choisi(s) en particulier parmi les groupes symbolisés par CH₃, C₂H₅, OH, OCH₃ et OCOCH₃ ;
- R¹, R² et R³ représentent chacun, indépendamment, un atome d'hydrogène ou un groupe alkyle en C₁-C₁₀ ;
- X et Y, indépendamment en chaque occurrence, ne représentent rien ou représentent un ou des chaînon(s) ou raccord(s) symbolisé(s) par -O-, -S-, -NR³-, -CO-, -CO-O-, -O-CO-, -O-CO-O-, -CO-NR³-, -NR³-CO-, -O-CO-NR³-, -NR³-CO-O-, ou -NR³-CO-NR³ ;
- l'indice m, indépendamment en chaque occurrence, peut prendre les valeurs de 2 à 6 ;
- et l'indice n, indépendamment en chaque occurrence, peut prendre les valeurs de 1 à 4.

2. Matériau dentaire conforme à la revendication 1, dans lequel, dans chaque cas indépendamment les uns des autres :
- A représente un chaînon symbolisé par -N=, ou un groupe de valence m et de type alkylène, cycloalkylène, hétérocycloalkylène, arylène, hétéroarylène, mixte alkylène-cyclyle ou cyclyl-oxy-cyclyle, étant entendu que :
le terme « alkylène » désigne, indépendamment dans chaque cas, un groupe aliphatique en C₁-C₂₀, linéaire ou ramifié, qui peut être interrompu par un ou plusieurs chaînon(s) ou raccord(s) symbolisé(s) par -O-, -S-, -CO-O-, -O-CO-, ou -O-CO-O-,
le terme « cycloalkylène » désigne, indépendamment dans chaque cas, un groupe cycloaliphatique en C₃-C₁₂,
le terme « hétérocycloalkylène » désigne, indépendamment dans chaque cas, un groupe hétérocycloaliphatique en C₃-C₁₂,
le terme « arylène » désigne, indépendamment dans chaque cas, un groupe aromatique en C₆-C₁₂,
le terme « hétéroarylène » désigne, indépendamment dans chaque cas, un groupe hétéroaromatique en C₃-C₁₂,
le terme « cyclyle » désigne, indépendamment dans chaque cas, un groupe de type cycloalkylène, hétérocycloalkylène, arylène ou hétéroarylène,
et les groupes indiqués ci-dessus peuvent porter chacun, indépendamment, un ou plusieurs substituant(s), choisi(s) en particulier parmi les groupes alkyle en C₁-C₃ et les groupes symbolisés par OCH₃ et OCOCH₃ ;
- CG représente, indépendamment en chaque occurrence, un groupe azoture N₃ ou un groupe alcyne choisi dans l'ensemble formé par les groupes de formule -CR¹R²-C≡CH, 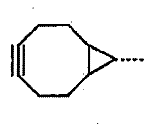 et 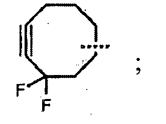
- Q, indépendamment en chaque occurrence, ne représente rien ou représente un groupe de valence n+1 et de type alkylène en C₁-C₁₀, aliphatique linéaire ou ramifié, qui peut être interrompu par un ou plusieurs chaînon(s) symbolisé(s) par -O- ou -S-, ou un groupe de type phénylène, étant entendu que les groupes indiqués ci-dessus peuvent porter chacun, indépendamment, un ou plusieurs substituant(s), choisi(s) en particulier parmi les groupes symbolisés par CH₃, C₂H₅, OCH₃ et OCOCH₃ ;
- R¹, R² et R³ représentent chacun, indépendamment, un atome d'hydrogène ou un groupe alkyle en C₁-C₅ et en particulier un groupe alkyle en C₁-C₃ ;
- X et Y, indépendamment en chaque occurrence, ne représentent rien ou représentent un ou des chaînon(s) ou raccord(s) symbolisé(s) par -O-, -S-, -CO-O-, -O-CO-, -O-CO-O-, -CO-NR³-, -NR³-CO-, -O-CO-NR³-, -NR³-CO-O-, ou -NR³-CO-NR³ ;
- l'indice m, indépendamment en chaque occurrence, peut prendre les valeurs 2, 3 ou 4 ;
- et l'indice n, indépendamment en chaque occurrence, peut prendre les valeurs 1, 2 ou 3, en particulier les valeurs 1 ou 2, et surtout la valeur 1.

3. Matériau dentaire conforme à la revendication 1 ou 2, qui contient au moins un azoture de formule IA :
A-[X-Q-(Y-CG)ₙ]ₘ Formule IA
dans laquelle
- A représente un chaînon ou raccord symbolisé par -O-, -N=, -NR³-, =N-N=, -NR³-N=, =N-NR³- ou -NR³-NR³-, ou un groupe de valence m et de type alkylène, cycloalkylène, hétérocycloalkylène, arylène, hétéroarylène, mixte alkylène-cyclyle ou cyclyl-oxy-cyclyle, étant entendu que :
le terme « alkylène » désigne, indépendamment dans chaque cas, un groupe aliphatique en C₁-C₅₀, linéaire ou ramifié, qui peut être interrompu par un ou plusieurs chaînon(s) ou raccord(s) symbolisé(s) par -O-, -S-, -NR³-, -CO-O-, -O-CO-, -O-CO-O-, -CO-NR³-, -NR³-CO-, -O-CO-NR³-, -NR³-CO-O-, ou -NR³-CO-NR³,
le terme « cycloalkylène » désigne, indépendamment dans chaque cas, un groupe cycloaliphatique en C₃-C₁₈,
le terme « hétérocycloalkylène » désigne, indépendamment dans chaque cas, un groupe hétérocycloaliphatique en C₃-C₁₈,
le terme « arylène » désigne, indépendamment dans chaque cas, un groupe aromatique en C₆-C₁₈,
le terme « hétéroarylène » désigne, indépendamment dans chaque cas, un groupe hétéroaromatique en C₃-C₁₈,
le terme « cyclyle » désigne, indépendamment dans chaque cas, un groupe de type cycloalkylène, hétérocycloalkylène, arylène ou hétéroarylène,
et les groupes indiqués ci-dessus peuvent porter chacun, indépendamment, un ou plusieurs substituant(s), choisi(s) en particulier parmi les groupes alkyle en C₁-C₅ et les groupes symbolisés par OH, OCH₃ et OCOCH₃ ;
- CG représente, indépendamment en chaque occurrence, un groupe azoture N₃ ;
- Q, indépendamment en chaque occurrence, ne représente rien ou représente un groupe de valence n+1 et de type alkylène en C₁-C₂₀, aliphatique linéaire ou ramifié, qui peut être interrompu par un ou plusieurs chaînon(s) symbolisé(s) par -O- ou -S-, ou un groupe de type phénylène, étant entendu que les groupes indiqués ci-dessus peuvent porter chacun, indépendamment, un ou plusieurs substituant(s), choisi(s) en particulier parmi les groupes symbolisés par CH₃, C₂H₅, OH, OCH₃ et OCOCH₃ ;
- R¹, R² et R³ représentent chacun, indépendamment, un atome d'hydrogène ou un groupe alkyle en C₁-C₁₀ ;
- X et Y, indépendamment en chaque occurrence, ne représentent rien ou représentent un ou des chaînon(s) ou raccord(s) symbolisé(s) par -O-, -S-, -NR³-, -CO-, -CO-O-, -O-CO-, -O-CO-O-, -CO-NR³-, -NR³-CO-, -O-CO-NR³-, -NR³-CO-O-, ou -NR³-CO-NR³ ;
- l'indice m, indépendamment en chaque occurrence, peut prendre les valeurs de 2 à 6 ;
- et l'indice n, indépendamment en chaque occurrence, peut prendre les valeurs de 1 à 4.
et au moins un alcyne de formule IB :
A-[X-Q-(Y-CG)ₙ]ₘ Formule IB
dans laquelle
- A représente un chaînon ou raccord symbolisé par -O-, -N=, -NR³-, =N-N=, -NR³-N=, =N-NR³- ou -NR³-NR³-, ou un groupe de valence m et de type alkylène, cycloalkylène, hétérocycloalkylène, arylène, hétéroarylène, mixte alkylène-cyclyle ou cyclyl-oxy-cyclyle, étant entendu que :
le terme « alkylène » désigne, indépendamment dans chaque cas, un groupe aliphatique en C₁-C₅₀, linéaire ou ramifié, qui peut être interrompu par un ou plusieurs chaînon(s) ou raccord(s) symbolisé(s) par -O-, -S-, -NR³-, -CO-O-, -O-CO-, -O-CO-O-, -CO-NR³-, -NR³-CO-, -O-CO-NR³-, -NR³-CO-O-, ou -NR³-CO-NR³,
le terme « cycloalkylène » désigne, indépendamment dans chaque cas, un groupe cycloaliphatique en C₃-C₁₈,
le terme « hétérocycloalkylène » désigne, indépendamment dans chaque cas, un groupe hétérocycloaliphatique en C₃-C₁₈,
le terme « arylène » désigne, indépendamment dans chaque cas, un groupe aromatique en C₆-C₁₈,
le terme « hétéroarylène » désigne, indépendamment dans chaque cas, un groupe hétéroaromatique en C₃-C₁₈,
le terme « cyclyle » désigne, indépendamment dans chaque cas, un groupe de type cycloalkylène, hétérocycloalkylène, arylène ou hétéroarylène,
et les groupes indiqués ci-dessus peuvent porter chacun, indépendamment, un ou plusieurs substituant(s), choisi(s) en particulier parmi les groupes alkyle en C₁-C₅ et les groupes symbolisés par OH, OCH₃ et OCOCH₃ ;
- CG représente, indépendamment en chaque occurrence, un groupe alcyne choisi dans l'ensemble formé par les groupes de formule -CR¹R²-C≡CH, 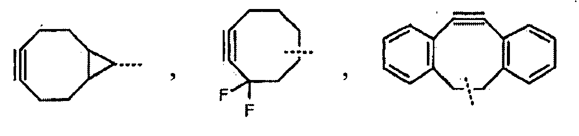 ou 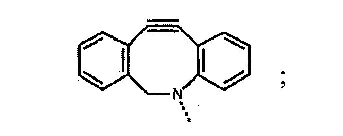
- Q, indépendamment en chaque occurrence, ne représente rien ou représente un groupe de valence n+1 et de type alkylène en C₁-C₂₀, aliphatique linéaire ou ramifié, qui peut être interrompu par un ou plusieurs chaînon(s) symbolisé(s) par -O- ou -S-, ou un groupe de type phénylène, étant entendu que les groupes indiqués ci-dessus peuvent porter chacun, indépendamment, un ou plusieurs substituant(s), choisi(s) en particulier parmi les groupes symbolisés par CH₃, C₂H₅, OH, OCH₃ et OCOCH₃ ;
- R¹, R² et R³ représentent chacun, indépendamment, un atome d'hydrogène ou un groupe alkyle en C₁-C₁₀ ;
- X et Y, indépendamment en chaque occurrence, ne représentent rien ou représentent un ou des chaînon(s) ou raccord(s) symbolisé(s) par
- O-, -S-, -NR³-, -CO-, -CO-O-, -O-CO-, -O-CO-O-, -CO-NR³-,
- NR³-CO-, -O-CO-NR³-, -NR³-CO-O-, ou -NR³-CO-NR³ ;
- l'indice m, indépendamment en chaque occurrence, peut prendre les valeurs de 2 à 6 ;
- et l'indice n, indépendamment en chaque occurrence, peut prendre les valeurs de 1 à 4.

4. Matériau dentaire conforme à l'une des revendications 1 à 3, dans lequel le composé de formule I présente une fonctionnalité moyenne supérieure à 2,2, et en particulier, supérieure à 2,5.

5. Matériau dentaire conforme à l'une des revendications 1 à 4, qui contient un catalyseur de cycloaddition azoture-alcyne.

6. Matériau dentaire conforme à la revendication 5, qui contient un catalyseur choisi dans l'ensemble constitué par les composés et les complexes de cuivre, ruthénium, nickel, palladium ou platine.

7. Matériau dentaire conforme à la revendication 5 ou 6, qui contient un composé du cuivre-II, en tant que catalyseur, et un agent réducteur photo-inductible.

8. Matériau dentaire conforme à la revendication 6 ou 7, qui contient un composé du cuivre-II choisi parmi ceux de formules CuSO₄, CuCl₂, CuBr₂, Cu(OH)₂, Cu(OAc)₂, CuO, Cu(ClO₄)₂ et Cu(NO₃)₂, ainsi que leurs hydrates et leurs produits d'addition de THF.

9. Matériau dentaire conforme à la revendication 7 ou 8, qui contient, en tant qu'agent réducteur photo-inductible, un photo-amorceur choisi dans l'ensemble formé par les photo-amorceurs de Norrish de type 1, les titanocènes et les alpha-dicétones.

10. Matériau dentaire conforme à l'une des revendications 1 à 9, qui contient un ou plusieurs monomère(s) polymérisable(s) par voie radicalaire.

11. Matériau dentaire conforme à la revendication 10, qui contient :
- du (méth)acrylate de méthyle, d'éthyle, d'hydroxy-éthyle, de butyle, de benzyle, de tétrahydrofurfuryle ou d'isobornyle, du méthacrylate de para-cumyl-phénoxy-éthylèneglycol (CMP-1E), du di(méth)acrylate de bisphénol A, du bis-GMA, du diméthacrylate de bisphénol A éthoxylé ou propoxylé, de l'UDMA, du di(méth)acrylate de diéthylèneglycol, triéthylèneglycol ou tétraéthylèneglycol, du tri(méth)-acrylate de triméthylol-propane, du tétra(méth)acrylate de pentaérythritol, du di(méth)acrylate ou tri(méth)acrylate de glycérol, du di(méth)acrylate de butane-1,4-diol, du di(méth)acrylate de décane-1,10-diol (D₃MA), du di(méth)acrylate de dodécane-1,12-diol,
- et/ou un ou plusieurs acrylamide(s) portant un ou deux N-substituant(s), du N-éthyl-acrylamide, du N,N-diméthyl-acrylamide, du N(2-hydroxy-éthyl)-acrylamide, du N-méthyl-N-(2-hydroxy-éthyl)-acrylamide, un ou plusieurs méthacrylamide(s) portant un seul N-substituant, du N-éthyl-méthacrylamide, du N-(2-hydroxy-éthyl)-méthacrylamide, de la N-vinyl-pyrrolidone, un ou plusieurs éther(s) d'allyle réticulant(s),
- et/ou une ou plusieurs pyrrolidone(s) réticulante(s), du 1,6-bis-(3-vinyl-pyrrolidon-2-yl)-hexane, un ou plusieurs bis-acrylamide(s) réticulant(s), du méthylène-bis(acrylamide), de l'éthylène-bis-(acryl-amide), un ou plusieurs bis(méth)acrylamide(s) réticulant(s), du N,N'-diéthyl-1,3-bis(acrylamido)-propane, du 1,3-bis(méthacryl-amido)-propane, du 1,4-bis(acrylamido)-butane, de la 1,4-bis-(acryloyl)-pipérazine,
ou un mélange de tels composés.

12. Matériau dentaire conforme à l'une des revendications 1 à 11, qui contient une ou des charge(s) organique(s) et/ou inorganique(s).

13. Matériau dentaire conforme à l'une des revendications 1 à 12, qui contient
a) de 0,1 à 80 % en poids, en particulier de 0,1 à 60 % en poids, de préférence de 5 à 50 % en poids, et mieux encore de 10 à 40 % en poids, d'au moins un composé de formule I, et de préférence, d'un mélange d'au moins un azoture de formule IA et d'au moins un alcyne de formule IB,
b) de 0,01 à 10 % en poids, de préférence de 0,1 à 3 % en poids, et mieux encore de 0,2 à 2 % en poids, d'un catalyseur,
c) de 0 à 80 % en poids, de préférence de 1 à 60 % en poids, et mieux encore de 5 à 50 % en poids, d'un co-monomère,
d) de 0 à 90 % en poids, de préférence de 10 à 80 % en poids, et mieux encore de 20 à 70 % en poids, d'une charge,
e) et de 0 à 70 % en poids, de préférence de 1 à 50 % en poids, et mieux encore de 5 à 20 % en poids, d'un solvant.

14. Utilisation d'un matériau dentaire conforme à l'une des revendications 1 à 13 pour la fabrication de composites dentaires ou de matériaux de restauration dentaire, et en particulier, pour la fabrication d'incrustations « inlay » ou « onlay », de couronnes, de bridges ou de matériaux d'obturation.

15. Utilisation d'au moins un composé de formule I, conforme à l'une des revendications 1 à 4, et en particulier, d'au moins un azoture de formule IA et d'au moins un alcyne de formule IB, conformes à la revendication 3 ou 4, pour la fabrication d'un matériau dentaire, et en particulier, d'un matériau composite dentaire.
